# EUROPEAN PATENT APPLICATION

(11) **EP 2 161 276 A2**
(43) Date of publication of application: **10.03.2010**
(21) Application number: 09014354.6
(22) Date of filing: 05.09.2003
(51) Int. Cl.: C07K 1/00, C07K 14/00

(54) **Methods, compositions and libraries pertaining PNA dimer and PNA oligomer systhesis**

(30) Priority: 08.09.2002 US 409220 P
(62) Divisional of application: 03755797.2
(71) Applicant: Applera Corporation, Norwalk, CT 06856-5435 (US)
(72) Inventor: Casale, Ralph A., Westford, MA 01886 (US); Coull, James M., Westford, MA 01886 (US); Dey, Subhakar, Wakefield, MA 01880 (US); Kowalczyk, Bruce A., Redwood City, CA 94061 (US); Gan, Kevin Z., Union City, CA 94587 (US)
(74) Representative: Schnappauf, Georg

(57) **Abstract**

This invention pertains to the field of PNA dimer and PNA oligomer synthesis.

## Description

### Disclosure Of The Invention

### I. List Of Certain Abbreviations Used Herein:

Fmoc = 9-fluorenylmethoxycarbonyl
Bhoc = benzhydroloxycarbonyl
Mmt = monomethoxytrityl
TFA = trifluoroacetic acid
boc or t-boc = tert-butoxycarbonyl
PAL = 5-(4'-aminomethyl-3',5'-dimethoxyphenoxy)valeric acid
MBHA = methylbenzhydrylamine
DHPP = 4-(1',1'-dimethyl-1'-hydroxypropyl)-phenoxyacetyl-alanyl-aminomethyl resin
DNA = 2'-deoxyribonucleic acid
RNA = ribonucleic acid
PNA = peptide nucleic acid
PEG = Polyethyleneglycol
DBU = 1,8-diazabicyclo-[5.4.0]-undec-7-ene
MeOH = methanol
ACN = acetonitrile

### II. Definitions:

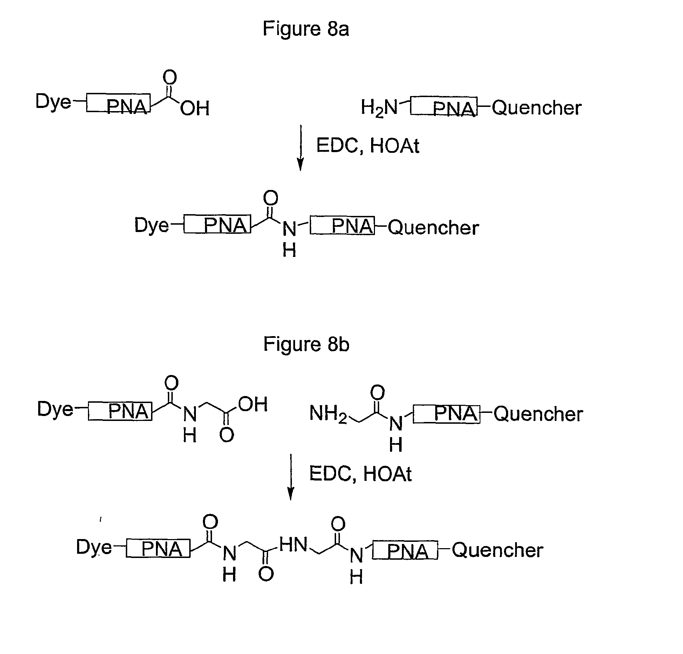

For the purposes of interpreting this specification the following definitions shall apply and whenever appropriate, terms used in the singular shall also include the plural and vice versa.
a. As used herein, "nucleobase" means those naturally occurring and those non-naturally occurring heterocyclic moieties commonly known to those who utilize nucleic acid technology or utilize peptide nucleic acid technology to thereby generate polymers that can sequence specifically bind to nucleic acids. Non-limiting examples of suitable nucleobases include: adenine, cytosine, guanine, thymine, uracil, 5-propynyl-uracil, 2-thio-5-propynyl-uracil, 5-methylcytosine, pseudoisocytosine, 2-thiouracil and 2-thiothymine, 2-aminopurine, N9-(2-amino-6-chloropurine), N9-(2,6-diaminopurine), hypoxanthine, N9-(7-deaza-guanine), N9-(7-deaza-8-aza-guanine) and N8-(7-deaza-8-aza-adenine). Other non-limiting examples of suitable nucleobases include those nucleobases illustrated in Figures 2(A) and 2(B) of Buchardt et al. (US 6,357,163).
b. As used herein, "nucleobase sequence" means any segment of nucleobase-containing subunits in an oligomer or polymer. Non-limiting examples of suitable oligomers or polymers include oligodeoxynucleotides (e.g. DNA), oligoribonucleotides (e.g. RNA), peptide nucleic acids (PNA), PNA chimeras, PNA oligomers, nucleic acid analogs and/or nucleic acid mimics.
c. As used herein, "target sequence" is a nucleobase sequence of a polynucleobase strand sought to be determined. It is to be understood that the nature of the target sequence is not a limitation. The polynucleobase strand comprising the target sequence may be provided from any source. For example, the target sequence may exist as part of a nucleic acid (e.g. DNA or RNA), PNA, nucleic acid analog or other nucleic acid mimic. The sample containing the target sequence may be provided from nature or it may be synthesized or supplied from a manufacturing process. When the target sequence is a subsequence of a nucleic acid, said nucleic acid can be obtained from any source. For example, said nucleic acid can be produced from a nucleic acid amplification process, contained in a cell or organism or otherwise be extracted from a cell or organism. Non-limiting examples of nucleic acid amplification processes that can be the source for the nucleic acid include, but are not limited to, Polymerase Chain Reaction (PCR), Ligase Chain Reaction (LCR), Strand Displacement Amplification (SDA), Transcription-Mediated Amplification (TMA), Q-beta replicase amplification (Q-beta) and Rolling Circle Amplification (RCA).
d. As used herein, "polynucleobase strand" means a single polymer strand comprising nucleobase-containing subunits. For example, a single nucleic acid strand of a double stranded nucleic acid is a polynucleobase strand.
e. As used herein, "nucleic acid" is a nucleobase sequence-containing oligomer or polymer, having a backbone formed from nucleotides, or analogs thereof. Preferred nucleic acids are DNA and RNA. For the avoidance of any doubt, PNA is a nucleic acid mimic and not a nucleic acid or nucleic acid analog.
f. As used herein, "peptide nucleic acid" or "PNA" means any oligomer or polymer comprising two or more PNA subunits (residues), including, but not limited to, any of the oligomer or polymer segments referred to or claimed as peptide nucleic acids in United States Patent Nos. 5,539,082, 5,527,675, 5,623,049, 5,714,331, 5,718,262, 5,736,336, 5,773,571, 5,766,855, 5,786,461, 5,837,459, 5,891,625 5,972,610, 5,986,053, 6,107,470 6,201,103, 6,228,982 and 6,357,163; all of which are herein incorporated by reference. The term "peptide nucleic acid" or "PNA" shall also apply to any oligomer or polymer segment comprising two or more subunits of those nucleic acid mimics described in the following publications: Lagriffoul et al., Bioorganic & Medicinal Chemistry Letters, 4: 1081-1082 (1994); Petersen et al., Bioorganic & Medicinal Chemistry Letters, 6: 793-796 (1996); Diderichsen et al., Tett. Lett. 37: 475-478 (1996); Fujii et al., Bioorg. Med. Chem. Lett. 7: 637-627 (1997); Jordan et al., Bioorg. Med. Chem. Lett. 7: 687-690 (1997); Krotz et al., Tett. Lett. 36: 6941-6944 (1995); Lagriffoul et al., Bioorg. Med. Chem. Lett. 4: 1081-1082 (1994); Diederichsen, U., Bioorganic & Medicinal Chemistry Letters, 7: 1743-1746 (1997); Lowe et al., J. Chem. Soc. Perkin Trans. 1, (1997) 1: 539-546; Lowe et al., J. Chem. Soc. Perkin Trans. 11: 547-554 (1997); Lowe et al., J. Chem. Soc. Perkin Trans. 1 1:5 55-560 (1997); Howarth et al., J. Org. Chem. 62: 5441-5450 (1997); Altmann, K-H et al., Bioorganic & Medicinal Chemistry Letters, 7: 1119-1122 (1997); Diederichsen, U., Bioorganic & Med. Chem. Lett., 8: 165-168 (1998); Diederichsen et al., Angew. Chem. Int. Ed., 37: 302-305 (1998); Cantin et al., Tett. Lett., 38: 4211-4214 (1997); Ciapetti et al., Tetrahedron, 53: 1167-1176 (1997); Lagriffoule et al., Chem. Eur. J., 3: 912-919 (1997); Kumar et al., Organic Letters 3(9):1269-1272 (2001); and the Peptide-Based Nucleic Acid Mimics (PENAMs) of Shah et al. as disclosed in WO96/04000.
   In certain embodiments, a "peptide nucleic acid" or "PNA" is an oligomer or polymer segment comprising two or more covalently linked subunits of the formula: 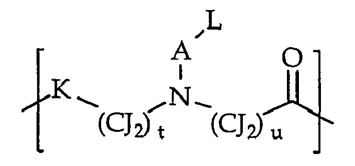 wherein, each J is the same or different and is selected from the group consisting of H, R¹, OR¹, SR¹, NHR¹, NR¹₂, F, Cl, Br and I. Each K is the same or different and is selected from the group consisting of O, S, NH and NR¹. Each R¹ is the same or different and is an alkyl group having one to five carbon atoms that may optionally contain a heteroatom or a substituted or unsubstituted aryl group. Each A is selected from the group consisting of a single bond, a group of the formula; -(CJ₂)ₛ- and a group of the formula; -(CJ₂)ₛC(O)-, wherein, J is defined above and each s is a whole number from one to five. Each t is 1 or 2 and each u is 1 or 2. Each L is the same or different and is independently selected from: adenine, cytosine, guanine, thymine, uracil, 5-propynyl-uracil, 2-thio-5-propynyl-uracil, 5-methylcytosine, pseudoisocytosine, 2-thiouracil and 2-thiothymine, 2-aminopurine, N9-(2-amino-6-chloropurine), N9-(2,6-diaminopurine), hypoxanthine, N9-(7-deaza-guanine), N9-(7-deaza-8-aza-guanine) and N8-(7-deaza-8-aza-adenine), other naturally occurring nucleobase analogs or other non-naturally occurring nucleobases.
   In certain other embodiments, a PNA subunit consists of a naturally occurring or non-naturally occurring nucleobase attached to the N-α-glycine nitrogen of the N-[2-(aminoethyl)]glycine backbone through a methylene carbonyl linkage; this currently being the most commonly used form of a peptide nucleic acid subunit.
g. As used herein, "terminal protecting group" means a protecting group covalently linked to the terminal nucleophilic functional group of a PNA monomer or PNA oligomer. For example, the terminal primary amine of a PNA monomer or oligomer that can be used in coupling a PNA monomer is the terminal nucleophilic functional group that is typically protected with the terminal protecting group.
h. As used herein, "nucleobase protecting group" means a protecting group covalently linked to a functional group of a nucleobase of a PNA monomer or oligomer to render the functional group unreactive during certain chemical reactions (e.g. ligation/condensation or coupling). For example, the exocylic amino groups of adenine, cytosine and guanine are typically protected with a suitable protecting group during the chemical assembly of a PNA oligomer. However, nucleobases, can be, but need not be, protected during the ligation/condensation reactions described herein.
i. As used herein, "PNA dimer" means two PNA subunits covalently linked together. The PNA dimer can be fully protected, partially protected or unprotected. By fully protected we mean that all of the reactive functional groups of the PNA dimer that are typically protected during solid phase chemical assembly of the PNA oligomer are protected with terminal protecting groups and/or nucleobase protecting groups. By partially protected we mean that at least one of the reactive functional groups of the PNA dimer that are typically protected during solid phase chemical assembly of a PNA oligomer does not comprise a protecting group. By unprotected we mean that all of the reactive functional groups of the PNA dimer that are usually protected during solid phase chemical assembly of the PNA oligomer do not comprise a protecting group. Examples of the functional groups of a PNA dimer that typically are protected with a protecting group, during solid phase chemical assembly, include the N-terminal amino group of the oligomer and the exocyclic amino groups of the nucleobases.
j. As used herein, "Fmoc(Bhoc) PNA monomer" or "Fmoc(Bhoc) monomer" means a PNA monomer comprising an Fmoc protecting group for protecting the N-terminal amine group and, where applicable, a Bhoc protecting group for protecting one or more of the exocyclic amine groups of the nucleobases, including without limitation, those Fmoc monomers commercially available from Applied Biosystems, Foster City, CA. For the avoidance of doubt, Fmoc(Bhoc) PNA monomer is intended to include the Fmoc thymine, Fmoc uracil, Fmoc 2-thiothymine or Fmoc 2-thiouracil monomers, despite the fact that these monomers do not possess an exocyclic amine group that requires a Bhoc protecting group.
k. As used herein, Mmt/Bhoc PNA monomer means a PNA monomer comprising an Mmt protecting group for protecting the N-terminal amine group and, where applicable, a Bhoc protecting group for protecting one or more of the exocyclic amine groups of the nucleobases (See Example 3). For the avoidance of doubt, Mmt/Bhoc PNA monomer is intended to include the Mmt thymine, Mmt uracil, Mmt 2-thiothymine or Mmt 2-thiouracil monomers, despite the fact that that these monomers do not possess an exocyclic amine group that requires a Bhoc protecting group.
l.As used herein, "PNA chimera" means an oligomer comprising two or more PNA subunits and one or more nucleic acid subunits (i.e. DNA or RNA), or analogs thereof, which are selected from different classes of subunits and that are linked by a covalent bond or a lanker. For example, a PNA/DNA chimera would comprise at least two PNA subunits covalently linked, via a chemical bond or linker, to at least one 2'-deoxyribonucleic acid subunit (For exemplary methods and compositions related to PNA/DNA chimera preparation See: WO96/40709).
m. As used herein, "acid forming cleavable linker" means a moiety attached to a solid support that cleavably links an oligomer or polymer (e.g. PNA) to said support during polymer chemical assembly and wherein that covalent bond can be cleaved by chemical treatment to thereby release the oligomer or polymer (generally after chemical assembly is completed) wherein the released polymer comprises an acid moiety at the point of its former attachment upon release from the solid support. For example, a PNA C-terminal acid oligomer is a PNA oligomer comprising a C-terminal acid group that is formed when the PNA oligomer is released from a solid support having an acid forming cleavable linker. For example, the C-terminal acid group of a PNA oligomer can be a C-terminal carboxylic acid or can be a C-terminal sulfonic acid.
n. As used herein, the terms "label", "reporter moiety" or "detectable moiety" are interchangeable and refer to moieties that can be attached to an oligomer or oligomer block, or otherwise can be used in a reporter system, to thereby render the oligomer detectable by an instrument or method. For example, a label can be any moiety that: (i) provides a detectable signal; (ii) interacts with a second label to modify the detectable signal provided by the first or second label; or (iii) confers a capture function, i.e. hydrophobic affinity, antibody/antigen, ionic complexation.
o. As used herein, "sequence specifically" means hybridization by base pairing through hydrogen bonding. Non-limiting examples of standard base pairing includes adenine base pairing with thymine or uracil and guanine base pairing with cytosine. Other non-limiting examples of base-pairing motifs include, but are not limited to: adenine base pairing with any of: 5-propynyl-uracil, 2-thio-5-propynyl-uracil, 2-thiouracil or 2-thiothymine; guanine base pairing with any of: 5-methylcytosine or pseudoisocytosine; cytosine base pairing with any of: hypoxanthine, N9-(7-deaza-guanine) or N9-(7-deaza-8-aza-guanine); thymine or uracil base pairing with any of: 2-aminopurine, N9-(2-amino-6-chloropurine) or N9-(2,6-diaminopurine); and N8-(7-deaza-8-aza-adenine), being a universal base, base pairing with any other nucleobase, such as for example any of: adenine, cytosine, guanine, thymine, uracil, 5-propynyl-uracil, 2-thio-5-propynyl-uracil, 5-methylcytosine, pseudoisocytosine, 2-thiouracil and 2-thiothymine, 2-aminopurine, N9-(2-amino-6-chloropurine), N9-(2,6-diaminopurine), hypoxanthine, N9-(7-deaza-guanine) or N9-(7-deaza-8-aza-guanine) (See: Seela et al., Nucl. Acids, Res.: 28(17): 3224-3232 (2000)).
p. As used herein, "condensation conditions" or "ligation conditions" means conditions suitable to condense/ligate two PNA oligomers in accordance with the condensation/ligation chemistry chosen.
q. As used herein "ligation" and "condensation" are interchangeable and refer to the process of covalently linking two oligomer blocks to thereby form an elongated PNA oligomer or chimera. It is also to be understood that the ligation/condensation chemistry is not to be a limitation of these methods. Non-limiting examples of numerous ligation/condensation chemistries suitable for forming elongated PNA oligomers are described herein with reference to Figures 8a and 8b.
r. As used herein, "quenching" means a decrease in fluorescence of a fluorescent reporter moiety caused by energy transfer associated with a quencher moiety, regardless of the mechanism.
s. As used herein "solid support" or "solid carrier" means any solid phase material upon which a PNA monomer, PNA dimer, PNA oligomer or PNA chimera is synthesized, attached, ligated or otherwise immobilized. Solid support encompasses terms such as "resin", "synthesis support", "solid phase", "surface" and/or "support". A solid support may be composed of organic polymers such as polystyrene, polyethylene, polypropylene, polyfluoroethylene, polyethyleneoxy, and polyacrylamide, as well as co-polymers and grafts thereof. A solid support may also be inorganic, such as glass, silica, controlled-pore-glass (CPG), or reverse-phase silica. The configuration of a solid support may be in the form of beads, spheres, particles, granules, a gel, or a surface. Surfaces may be planar, substantially planar, or non-planar. Solid supports may be porous or non-porous, and may have swelling or non-swelling characteristics. A solid support may be configured in the form of a well, depression or other container, vessel, feature or location. A plurality of solid supports may be configured in an array at various locations, addressable for robotic delivery of reagents, or by detection means including scanning by laser illumination and confocal or deflective light gathering.
t. As used herein, "sterically hindered solid support" means a solid support comprising a sterically hindered cleavable linker or sterically hindered acid forming cleavable linker. By sterically hindered we mean that the linker comprises a secondary or tertiary atom that forms the covalent cleavable bond between the linker and the oligomer that is assembled on the solid support. Non-limiting examples of sterically hindered solid supports include: Trityl chloride resin (trityl-Cl, Novabiochem, P/N 01-64-0074), 2-Chlorotrityl chloride resin (Novabiochem, P/N 01-64-0021), DHPP (Bachem, P/N Q-1755), MBHA (Applied Biosystems P/N 400377), 4-methyltrityl chloride resin (Novabiochem, P/N 01-64-0075), 4-methoxytrityl chloride resin (Novabiochem, P/N 01-64-0076), Hydroxy-(2-chorophnyl)methyl-PS (Novabiochem, P /N 01-64-0345), Rink Acid Resin (Novabiochem P/Ns 01-64-0380, 01-64-0202), NovaSyn TGT alcohol resin (Novabiochem, P/N 01-64-0074).
u. As used herein, "support bound" means immobilized on or to a solid support.
v. As used herein "array" or "microarray" means a predetermined spatial arrangement of oligomers present on a solid support or in an arrangement of vessels. Certain array formats are referred to as a "chip" or "biochip" (M. Schena, Ed. Microarray Biochip Technology, BioTechnique Books, Eaton Publishing, Natick, MA (2000). An array can comprise a low-density number of addressable locations, e.g. 2 to about 12, medium-density, e.g. about a hundred or more locations, or a high-density number, e.g. a thousand or more. Typically, the array format is a geometrically regular shape that allows for fabrication, handling, placement, stacking, reagent introduction, detection, and storage. The array may be configured in a row and column format, with regular spacing between each location. Alternatively, the locations may be bundled, mixed, or homogeneously blended for equalized treatment or sampling. An array may comprise a plurality of addressable locations configured so that each location is spatially addressable for high-throughput handling, robotic delivery, masking, or sampling of reagents, or by detection means including scanning by laser illumination and confocal or deflective light gathering.
w. As used herein, "block", "oligomer block" or "block oligomer" are interchangeable and all mean a PNA oligomer or PNA chimera that is designed and available to be ligated to a second appropriately modified PNA oligomer or chimera to thereby prepare an elongated oligomer. Oligomers or blocks that are ligated/condensed may be unlabeled, labeled with one or more reporter moieties and/or comprise one or more protected or unprotected functional groups. With respect to an elongated oligomer, block can also be used to refer to a part of the elongated oligomer that originates from a block used to form the elongated oligomer. The elongated oligomer also may be used as a block in a ligation/condensation reaction that further elongates the oligomer.
x. As used herein, polymer and oligomer are essentially interchangeable when referring to a PNA oligomer or PNA chimera of two or more subunits in length.
y. As used herein, "native oligomer" means PNA oligomer or PNA chimera that does not comprise a linker that separates two oligomer blocks of an elongated oligomer. Thus, a native oligomer, even if a chimera, comprises a PNA backbone that is unmodified at the point of ligation and is therefore indistinguishable from that which would be produced using denovo chemical assembly of PNA monomers.

### III. General:

### PNA Oligomer Synthesis Through Chemical Assembly:

Methods for the chemical assembly of PNAs are well known (See: Patent Nos. 5,539,082, 5,527,675, 5,623,049, 5,714,331, 5,718,262, 5,736,336, 5,773,571, 5,766,855, 5,786,461, 5,837,459, 5,891,625, 5,972,610, 5,986,053, 6,107,470, 6,201,103, 6,228,982 and 6,357,163; all of which are herein incorporated by reference (Also see: PerSeptive Biosystems Product Literature)). As a general reference for PNA synthesis methodology also please see: Nielsen et al., Peptide Nucleic Acids; Protocols and Applications, Horizon Scientific Press, Norfolk England (1999).

Chemicals and instrumentation for the support bound automated chemical assembly of peptide nucleic acids are now commercially available. Both labeled and unlabeled PNA oligomers are likewise available from commercial vendors of custom PNA oligomers. Chemical assembly of a PNA is analogous to solid phase peptide synthesis, wherein at each cycle of assembly the oligomer possesses a reactive alkyl amino terminus that is condensed with the next synthon to be added to the growing polymer.

PNA may be synthesized at any scale, from submicromole to millimole, or more. PNA can be conveniently synthesized at the 2 µmole scale, using Fmoc(Bhoc), tBoc/Z, or MmT protecting group monomers on an Expedite Synthesizer (Applied Biosystems) using a XAL or PAL support. Alternatively the Model 433A Synthesizer (Applied Biosystems) with MBHA support can be used. Moreover, many other automated synthesizers and synthesis supports can be utilized. Because standard peptide chemistry is utilized, natural and non-natural amino acids can be routinely incorporated into a PNA oligomer. Because a PNA is a polyamide, it has a C-terminus (carboxyl terminus) and an N-terminus (amino terminus). For the purposes of the design of a hybridization probe suitable for antiparallel binding to the target sequence (the preferred orientation), the N-terminus of the probing nucleobase sequence of the PNA probe is the equivalent of the 5'-hydroxyl terminus of an equivalent DNA or RNA oligonucleotide.

### PNA Oligomer Synthesis Through Ligation/Condensation

When used in ligation/condensation reactions, the nature of the ligation chemistry chosen should be considered. For simplicity, we sometimes refer to one of the oligomers used in a ligation/condensation reaction as a terminal oligomer or terminal block and the other as the condensation oligomer or condensation block. This distinction is generally irrelevant except to distinguish between the different blocks especially if they contain the same nucleobase sequence. Often at least the nature of the functional groups that are used in the ligation will be different for the terminal and condensation oligomer blocks since they can be designed to accommodate different ligation chemistries. For example, one of the oligomer blocks can comprise a C-terminal acid group and the other can comprise an N-terminal amine group wherein the product of the condensation/ligation reaction is an amide bond that forms an elongated PNA oligomer or chimera.

However, when the oligomer is to be extended by multiple ligations, we will generally refer to the terminal oligomer block as the oligomer block produced from the first ligation or from the immediately preceding ligation step. Several non-limiting examples of ligation chemistries are illustrated in Figures 8a & 8b. Using no more than routine experimentation as well as the description contained herein, one of ordinary skill in the art will easily be able to prepare elongated PNA oligomers or PNA chimeras.

The terminal blocks may comprise a C-terminal amide that is relatively unreactive. In contrast, the condensing blocks may comprise a C-terminal end that is suitable for the ligation reaction. However, depending upon the nature of the condensation chemistry, the C-terminal end of the oligomer may comprise a C-terminal acid. If a functional group, the termini of an oligomer to by ligated/condensed may or may not require the addition of a terminal protecting group depending on the nature of the condensation/ligation chemistry. Since the oligomer blocks are themselves often prepared by de novo methods and because suitable commercial reagents and instrumentation are available for the production of PNA oligomers comprising a C-terminal amino acid or C-terminal amide, one of skill in the art can easily prepare the oligomer blocks of the desired C-terminal configuration.

With respect to the N-terminus, again the exact configuration can depend on the nature of the ligation chemistry chosen and on whether or not the oligomer is a condensing oligomer block or a terminal oligomer block. If the oligomer is a terminal block, the N-terminus may comprise a reactive functional group (e.g. N-terminal amine group) whereas if the oligomer is a condensing oligomer block, the N-terminus can be capped. Non-limiting examples of capping include labeling the N-terminus with a label or otherwise reacting it with a relatively non-reactive moiety such as acetyl. If the N-terminus is to be involved in the ligation reaction, it will typically exist as a free amine. Since the oligomer blocks are themselves prepared by de novo methods and because suitable commercial reagents and instrumentation are available for the production of PNA oligomers, one of skill in the art can easily prepare the oligomer blocks of the desired N-terminal configuration.

In addition to the modification of the termini for ligation, the oligomer blocks can be modified and/or properly protected to thereby incorporate functional groups for labeling or for attachment to surfaces. Such functional groups can be utilized either before or after ligation depending upon factors such as:1) the oligomer synthesis chemistry (e.g. harsh deprotection conditions required that might destroy a label), the condensation/ligation chemistry chosen (e.g. functional groups of the desired label might interfere with the condensation chemistry) and the intended use of the functional group (e.g. whether it is intended for labeling or for attachment to a solid support).

### PNA Labeling/Modification:

Non-limiting methods for labeling PNAs are described in US 6,110,676, US 6,280,964, US 6,355,421, WO99/21881, US 6,361,942, WO99/49293 and US 6,441,152 (all of which are herein incorporated by reference), the examples section of this specification or are otherwise well known in the art of PNA synthesis and peptide synthesis. Methods for labeling PNA are also discussed in Nielsen et al., Peptide Nucleic Acids; Protocols and Applications, Horizon Scientific Press, Norfolk, England (1999). Non-limiting methods for labeling PNA oligomers are discussed below.

Because the synthetic chemistry of assembly is essentially the same, any method commonly used to label a peptide can often be adapted to effect the labeling a PNA oligomer. Generally, the N-terminus of the oligomer or polymer can be labeled by reaction with a moiety having a carboxylic acid group or activated carboxylic acid group. One or more spacer moieties can optionally be introduced between the labeling moiety and the nucleobase containing subunits of the oligomer. Generally, the spacer moiety can be incorporated prior to performing the labeling reaction. If desired, the spacer may be embedded within the label and thereby be incorporated during the labeling reaction.

Typically the C-terminal end of the polymer can be labeled by first condensing a labeled moiety or functional group moiety with the support upon which the PNA oligomer is to be assembled. Next, the first nucleobase containing synthon of the PNA oligomer can be condensed with the labeled moiety or functional group moiety. Alternatively, one or more spacer moieties (e.g. 8-amino-3,6-dioxaoctanoic acid; the "O-linker") can be introduced between the label moiety or functional group moiety and the first nucleobase subunit of the oligomer. Once the molecule to be prepared is completely assembled, labeled and/or modified, it can be cleaved from the support deprotected and purified using standard methodologies.

For example, the labeled moiety or functional group moiety can be a lysine derivative
wherein the ε-amino group is a protected or unprotected functional group or is otherwise modified with a reporter moiety. The reporter moiety could be a fluorophore such as 5(6)-carboxyfluorescein, Dye1, Dye2 or a quencher moiety such as 4-((4-(dimethylamino)phenyl)azo)benzoic acid (dabcyl). Condensation of the lysine derivative with the solid support can be accomplished using standard condensation (peptide) chemistry. The α-amino group of the lysine derivative can then be deprotected and the nucleobase sequence assembly initiated by condensation of the first PNA synthon with the α-amino group of the lysine amino acid. As discussed above, a spacer moiety may optionally be inserted between the lysine amino acid and the first PNA synthon by condensing a suitable spacer (e.g. Fmoc-8-amino-3,6-dioxaoctanoic acid) with the lysine amino acid prior to condensation of the first PNA synthon.

Alternatively, a functional group on the assembled, or partially assembled, polymer can be introduced while the oligomer is still support bound. The functional group will then be available for any purpose, including being used to either attached the oligomer to a support or otherwise be reacted with a reporter moiety, including being reacted post-ligation (by post-ligation we mean at a point after the oligomer has been fully formed by the performing of one or more condensation/ligation reactions). This method, however, requires that an appropriately protected functional group be incorporated into the oligomer during assembly so that after assembly is completed, a reactive functional can be generated. Accordingly, the protected functional group can be attached to any position within the oligomer or block, including, at the oligomer termini, at a position internal to the oligomer.

For example, the ε-amino group of a lysine could be protected with a 4-methyl-triphenylmethyl (Mtt), a 4-methoxy-triphenylmethyl (MMT) or a 4,4'-dimethoxytriphenylmethyl (DMT) protecting group. The Mtt, MMT or DMT groups can be removed from the oligomer (assembled using commercially available Fmoc PNA monomers and polystyrene support having a PAL linker; PerSeptive Biosystems, Inc., Framingham, MA) by treatment of the synthesis resin under mildly acidic conditions. Consequently, a donor moiety, acceptor moiety or other reporter moiety, for example, can then be condensed with the ε-amino group of the lysine amino acid while the polymer is still support bound. After complete assembly and labeling, the polymer can then cleaved from the support, deprotected and purified using well-known methodologies.

By still another method, the reporter moiety can be attached to the oligomer or oligomer block after it is fully assembled and cleaved from the support. This method is preferable where the label is incompatible with the cleavage, deprotection or purification regimes commonly used to manufacture the oligomer. By this method, the PNA oligomer can be labeled in solution by the reaction of a functional group on the polymer and a functional group on the label. Those of ordinary skill in the art will recognize that the composition of the coupling solution will depend on the nature of oligomer and label, such as for example a donor or acceptor moiety. The solution may comprise organic solvent, water or any combination thereof. Generally, the organic solvent will be a polar non-nucleophilic solvent. Non limiting examples of suitable organic solvents include acetonitrile (ACN), tetrahydrofuran, dioxane, methyl sulfoxide, N,N'-dimethylformamide (DMF) and 1-methyl pyrrolidone (NMP).

The functional group on the polymer to be labeled can be a nucleophile (e.g. an amino group) and the functional group on the label can be an electrophile (e.g. a carboxylic acid or activated carboxylic acid). It is however contemplated that this can be inverted such that the functional group on the polymer can be an electrophile (e.g. a carboxylic acid or activated carboxylic acid) and the functional group on the label can be a nucleophile (e.g. an amino acid group). Non-limiting examples of activated carboxylic acid functional groups include N-hydroxysuccinimidyl esters. In aqueous solutions, the carboxylic acid group of either of the PNA or label (depending on the nature of the components chosen) can be activated with a water soluble carbodiimide. The reagent, 1-ethyl-3-(3-dimethylamino-propyl)carbodiimide hydrochloride (EDC), is a commercially available reagent sold specifically for aqueous amide forming condensation reactions. Such condensation reactions can also be improved when 1-Hydroxy-7-azabenzotriazole (HOAt) or 1-hydrozybenzotriazole (HOBt) is mixed with the EDC.

The pH of aqueous solutions can be modulated with a buffer during the condensation reaction. For example, the pH during the condensation can be in the range of 4-10. Generally, the basicity of non-aqueous reactions will be modulated by the addition of non-nucleophilic organic bases. Non-limiting examples of suitable bases include N-methylmorpholine, triethylamine and N,N-diisopropylethylamine. Alternatively, the pH can be modulated using biological buffers such as (N-[2-hydroxyethyl]piperazine-N'-[2-ethanesulfonic acid) (HEPES) or 4-morpholineethane-sulfonic acid (MES) or inorganic buffers such as sodium bicarbonate.

### PNA Chimera Synthesis And Labeling/Modification:

PNA chimeras are a combination of a nucleic acid and peptide nucleic acid subunits. Hence, the synthesis, labeling and modification of PNA chimeras can utilize methods known to those of skill in the art as well as those described above. A suitable reference for the synthesis, labeling and modification of PNA chimeras can be found in WIPO published patent application number WO96/40709, now issued as US Patent No. 6,063,569, herein incorporated by reference. Moreover, the methods described above for PNA synthesis and labeling often can be used for modifying the PNA portion of a PNA chimera. Additionally, well-known methods for the synthesis and labeling of nucleic acids can often be used for modifying the nucleic acid portion of a PNA chimera. Exemplary methods can be found in 5,476,925, 5,453,496, 5,446,137, 5,419,966, 5,391,723, 5,391,667, 5,380,833, 5,348,868, 5,281,701, 5,278,302, 5,262,530, 5,243,038, 5,218,103, 5,204,456, 5,204,455, 5,198, 540, 5,175,209, 5,164,491, 5,112,962, 5,071,974, 5,047,524, 4,980,460, 4,923,901, 4,786,724, 4,725,677, 4,659,774, 4,500,707, 4,458,066, and 4,415,732; all of which are herein incorporated by reference.

### Labeled Oligomers & Oligomer Blocks:

As discussed above, PNA chimeras and PNA oligomers can be labeled with reporter moieties. Non-limiting examples of reporter moieties (labels) suitable for directly labeling oligomers or oligomer blocks include: a quantum dot, a minor groove binder, a dextran conjugate, a branched nucleic acid detection system, a chromophore, a fluorophore, a quencher, a spin label, a radioisotope, an enzyme, a hapten, an acridinium ester and a chemiluminescent compound. Quenching moieties are also considered labels. Other suitable labeling reagents and preferred methods of attachment would be recognized by those of ordinary skill in the art of PNA, peptide or nucleic acid synthesis. Non-limiting examples are described or referred to above.

Non-limiting examples of haptens include 5(6)-carboxyfluorescein, 2,4-dinitrophenyl, digoxigenin, and biotin.

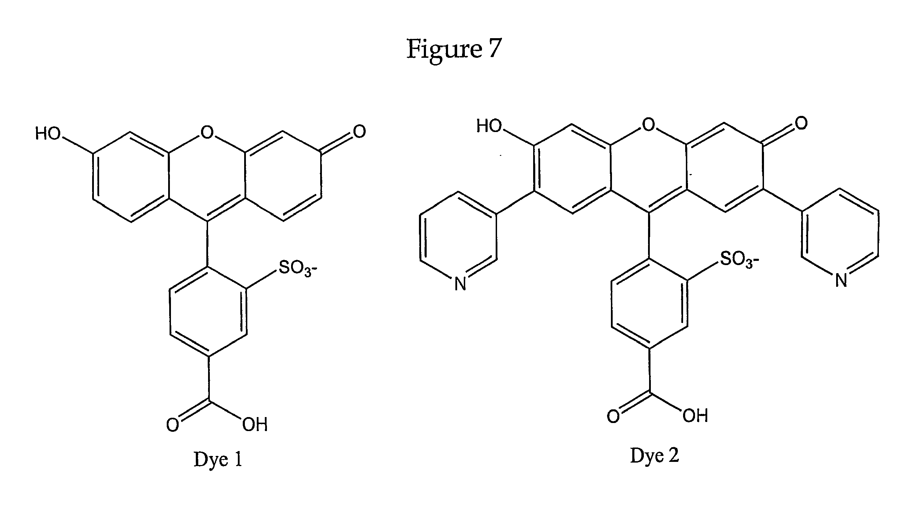

Non-limiting examples of fluorochromes (fluorophores) include 5(6)-carboxyfluorescein (Flu), 2',4',1,4,-tetrachlorofluorescein; and 2',4',5',7',1,4-hexachlorofluorescein, other fluorescein dyes (See: U.S. Patent Nos. 5,188,934; 6,008,379; 6,020,481, incorporated herein by reference), 6-((7-amino-4-methylcoumarin-3-acetyl)amino)hexanoic acid (Cou), 5(and 6)-carboxy-X-rhodamine (Rox), other rhodamine dyes (See: U.S. Patent Nos. 5,366,860; 5,847,162; 5,936,087; 6,051,719; 6,191,278; 6,248,884, incorporated herein by reference), benzophenoxazines (See: U.S. Patent No. 6,140,500, incorporated herein by reference)Cyanine 2 (Cy2) Dye, Cyanine 3 (Cy3) Dye, Cyanine 3.5 (Cy3.5) Dye, Cyanine 5 (Cy5) Dye, Cyanine 5.5 (Cy5.5) Dye Cyanine 7 (Cy7) Dye, Cyanine 9 (Cy9) Dye (Cyanine dyes 2, 3, 3.5, 5 and 5.5 are available as NHS esters from Amersham, Arlington Heights, IL), other cyanine dyes (Kubista, WO 97/45539), 6-carboxy-4',5'-dichloro-2',7'-dimethoxyfluorescein (JOE), 5(6)-carboxy-tetramethyl rhodamine (Tamara), Dye 1 (Figure 7), Dye2 (Figure 7) or the Alexa dye series (Molecular Probes, Eugene, OR).

Non-limiting examples of enzymes include polymerases (e.g. Taq polymerase, Klenow PNA polymerase, T7 DNA polymerase, Sequenase, DNA polymerase 1 and phi29 polymerase), alkaline phosphatase (AP), horseradish peroxidase (HRP), soy bean peroxidase (SBP)), ribonuclease and protease.

Non-limiting examples of quenching moieties include diazo-containing moieties such as aryldiazo compounds, e.g. dabcyl and dabsyl, homologs containing one more additional diazo and/or aryl groups; e.g. Fast Black, (Nardone, U.S. Patent No. 6,117,986), and substituted compounds where Z is a substituent such Cl, F, Br, C₁-C₆ alkyl, C₅-C₁₄ aryl, nitro, cyano, sulfonate, NR₂, -OR, and CO₂H, where each R is independently H, C₁-C₆ alkyl or C₅-C₁₄ aryl according to the structures: 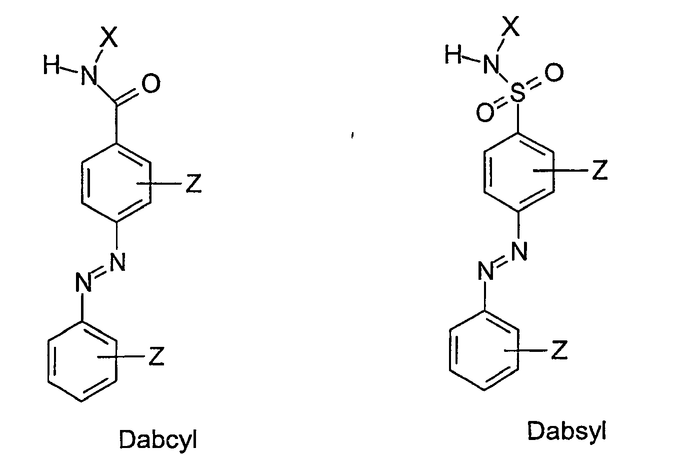 cyanine dyes (Lee, US Patent No. 6,080,868), including the exemplary structure: 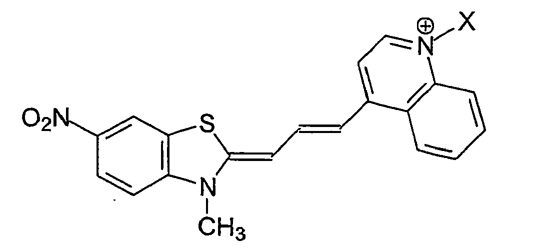 and other chromophores such as anthraquinone, malachite green, nitrothiazole, and nitroimidazole compounds and the like wherein the group X is the covalent attachment site of a bond or linker to the oligomers of the invention.

A non-limiting example of a minor groove binder is CDPI₃, represented by the structure: 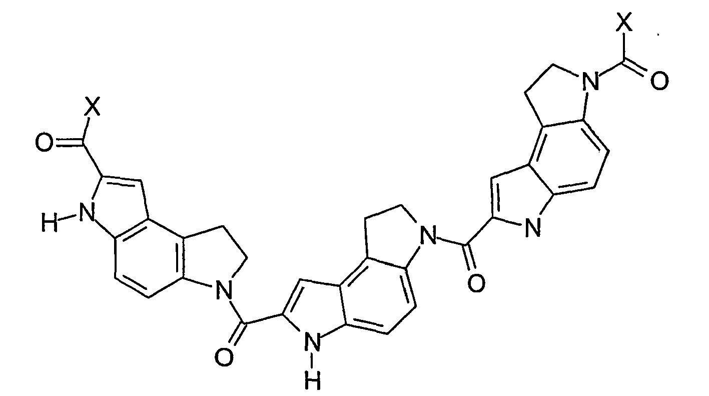 where X are exemplary attachment sites to a oligomer (Dempcy, WO 01/31063).

Non-radioactive labeling methods, techniques, and reagents are reviewed in: *Non-*Radioactive Labeling, A Practical Introduction, Garman, A.J. Academic Press, San Diego, CA (1997)

### Spacer/Linker Moieties:

Generally, spacers can be used to minimize the adverse effects that bulky labeling reagents might have on the hybridization properties of probes or primers. A linker can be used to link two or more oligomer blocks of an oligomer. The linkers can be abasic. By abasic we mean that they do not comprise a nucleobase. Non-limiting examples of spacer/linker moieties are: one or more aminoalkyl carboxylic acids (e.g. aminocaproic acid), the side chain of an amino acid (e.g. the side chain of lysine or ornithine), one or more natural amino acids (e.g. glycine), aminooxyalkylacids (e.g. 8-amino-3,6-dioxaoctanoic acid), alkyl diacids (e.g. succinic acid), alkyloxy diacids (e.g. diglycolic acid) or alkyldiamines (e.g. 1,8-diamino-3,6-dioxaoctane). Spacer/linker moieties may also incidentally or intentionally be constructed to improve the water solubility of the oligomer (For example see: Gildea et al., Tett. Lett. 39: 7255-7258 (1998)). Guidance In Label Choices When Ligating/Condensing

### Oligomer Blocks:

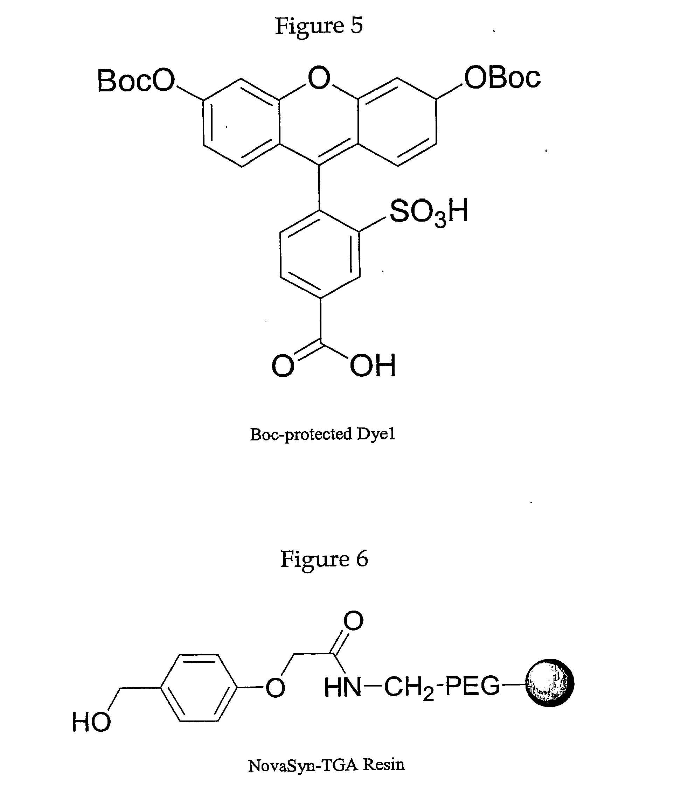

It will be apparent to one of skill in the art that when oligomers are to be condensed/ligated, to thereby produce an elongated oligomer, the entire nature of the potentially reactive functional groups of the component oligomer blocks should be considered for potential side or cross-reactions. Protecting groups can also be used, as appropriate, to minimize or eliminate potential side or cross-reactions. For example, when labeled oligomers are to be ligated, it is wise to consider the potential for reactivity of functional groups of the one or more labels in view of the nature of the various ligation chemistries that can be chosen. Alternatively, protected labels can be used (See for Example, Figure 5).

### Non-limiting Examples of Ligation/Condensation Chemistries

With reference to Figures 8a and 8b, properly prepared oligomer blocks can be ligated using a carbodiimide, such as the water-soluble carbodiimide 1-Ethyl-3-(3-Dimethylaminopropyl)carbodiimide hydrochloride (EDC). As illustrated, typically one of the oligomer blocks comprises a carboxylic acid moiety and the other comprises an amine group. Because PNA oligomers, whether or not they comprise linked natural amino acid moieties, can comprise an amine terminus and a carboxylic acid terminus, generally PNA oligomer blocks do not require modification to facilitate this type ligation chemistry; except for the preparation of at least one PNA C-terminal acid oligomer instead of the more typical C-terminal acid. The oligomers can be ligated in an aqueous solution, optionally containing up to about 75 percent organic modifier (v/v). The pH can be less than 6.5. The addition of an activating reagent such as a triazole compound (e.g. 1-Hydroxy-7-azabenzotriazole (HOAt) or 1-Hydroxybenzotriazole (HOBt)) can increase the overall yield of the condensation/ligation reaction.

With reference to Figures 8a and 8b, the product of the ligation/condensation is illustrated as comprising both a donor and acceptor moiety. This however is an example and not a limitation as one or both of the oligomers to be ligated can be unlabeled. Conveniently, this ligation/condensation process can produce a native oligomer using the PNA C-terminal acid oligomers that comprise a C-terminal PNA subunit as described herein.

In one embodiment, conveniently these native oligomers, if labeled as a Linear Beacon as described in more detail in copending and commonly owned USSN 09 / 179,162 (incorporated herein by reference), can be used for the analysis of target sequences, including in multiplex SNP genotyping assays. Accordingly, the compositions, methods and libraries of this invention can be used in the production, through a library approach, of native PNA oligomers that can be used for many purposes.

### Other

United States published application 2003-0077608 and PCT published application WO02/072865 are copending and commonly owed with this application. Said published patent applications describe, *inter alia,* the ligation of oligomer blocks wherein there is a linker of at least three atoms that separates the blocks in the elongated (combination) oligomer. Accordingly, the ligation/condensation reactions described in these published patent applications do not produce native oligomers as describe herein. Nevertheless, said application describes various methods for condensing/ligating oligomer blocks that can be applied to the PNA C-terminal acid oligomers described herein. Condensation/ligation of the PNA C-terminal acid oligomers can be used to produce native PNA oligomers. Because they are native PNA oligomers, they can be used for any application commonly applied to PNA oligomers including, for example, multiplex SNP genotyping using self-indicating PNA probes.

### IV. Embodiments Of The Invention:

### a) Introduction

This invention pertains to the field of PNA dimer and PNA oligomer synthesis. The PNA dimers, including libraries of the dimers whether or not support bound, can, *inter alia,* be used in the preparation of PNA C-terminal acid oligomers. Furthermore, the PNA C-terminal acid oligomers can themselves, *inter alia,* be used in the preparation of longer PNA oligomers and/or chimeras through ligation/condensation as well as be used, *inter alia,* in the preparation of libraries used in PNA oligomer and/or chimera preparation. The PNA oligomers and PNA chimera so produced can themselves, *inter alia,* be used in the determination of target sequences of interest, including without limitation, by use in self-indicating assays, multiplex assays and/or self-indicating multiplex assays.

### b) Support Bound PNA Dimer Compositions

In some embodiments, this invention pertains to a solid support composition. The solid support comprises an acid forming cleavable linker and a PNA dimer. The PNA dimer comprises an N-terminal base labile protecting group and is cleavably linked to the solid support through the cleavable linker. Furthermore, the loading of the PNA dimer on the solid support can be greater than or equal to 0.08 mmol per gram. The PNA dimer can be formed from Fmoc(Bhoc) monomers. The PNA dimer can be linked to the cleavable linker of the solid support by an ester bond.

The solid support can be a sterically hindered solid support. Non-limiting examples of sterically hindered solid supports include: Trityl chloride resin (Trityl-Cl), 2-Chlorotrityl chloride resin, DHPP, MBHA, 4-methyltrityl chloride resin, 4-methoxytrityl chloride resin, Hydroxy-(2-chorophenyl)methyl-PS, Rink Acid Resin and NovaSyn TGT alcohol resin. The solid support can also be selected from the group consisting of: PAL-PEG-PS™, NovaSyn TGA and Wang Resin.

The loading of the PNA dimer on the solid support can be in the range from about 0.1 mmol per gram to about 1 mmol per gram. The loading of the PNA dimer on the solid support can be in the range from about 0.12 mmol per gram to about 0.35 mmol per gram.

The support bound PNA dimers can be arranged on the support to produce an array comprising two or more different support bound PNA dimers.

The support bound PNA dimers can be produced by a variety of methods and or PNA monomer types, including without limitation, the methods described in Section IV(d) or IV(e), below.

### c) A Library Of Support Bound PNA Dimer Solid Supports

In some embodiments, this invention pertains to a library of solid supports. The library comprises at least two solid supports wherein the at least two solid supports each comprise an acid forming cleavable linker and a PNA dimer. The PNA dimer can be cleavably linked to the acid forming cleavable linker. The PNA dimer can differ in nucleobase sequence from the PNA dimer that is linked to any of the other of the at least two solid supports of the library. The PNA dimer can be linked to the cleavable linker of the solid support by an ester bond.

The library can comprise at least sixteen solid supports. For example, each support can comprise a PNA dimer chosen from a set of at least sixteen possible PNA dimers wherein each PNA dimer of the set differs from all of the other PNA dimers of the set by at least one of at least four different nucleobases of the PNA subunits used in the assembly of the PNA dimers. The at least four different nucleobases can be selected from the group consisting of: adenine, cytosine, guanine, thymine, uracil, 5-propynyl-uracil, 2-thio-5-propynyl-uracil, 5-methylcytosine, pseudoisocytosine, 2-thiouracil and 2-thiothymine, 2-aminopurine, N9-(2-amino-6-chloropurine), N9-(2,6-diaminopurine), hypoxanthine, N9-(7-deaza-guanine), N9-(7-deaza-8-aza-guanine) and N8-(7-deaza-8-aza-adenine). For example, see the library of 16 solid supports described in Example 1.

The solid supports of the library can be a sterically hindered solid support. For example, the sterically hindered solid support can be selected from the group consisting of: Trityl chloride resin (Trityl-Cl), 2-Chlorotrityl chloride resin, DHPP, MBHA, 4-methyltrityl chloride resin, 4-methoxytrityl chloride resin, Hydroxy-(2-chorophenyl)methyl-PS, Rink Acid Resin and NovaSyn TGT alcohol resin. The solid support can also be selected from the group consisting of: PAL-PEG-PS™, NovaSyn TGA and Wang Resin.

The C-terminal subunit of the PNA dimers of the solid supports of the library can be linked to the cleavable linker. The PNA dimers can be formed from Fmoc(Bhoc) protected monomers. The PNA dimers can be formed from t-boc/Z protected monomers. The PNA dimers can be formed from Mmt/Bhoc protected monomers. The PNA dimers can be formed from other types of PNA monomers or a combination of different types of PNA monomers (See: Example 3, below). Accordingly, it is clear that the library of PNA dimer solid supports can be produced by a variety of methods and/or PNA monomer types, including without limitation, the method described in Section V(d) or V(e), below.

The loading of the PNA dimer on at least one solid support of the library can be greater than or equal to 0.08 mmol per gram. The loading of the PNA dimer on at least one half of the solid supports of the library can be greater than or equal to 0.08 mmol per gram. The loading of the PNA dimer on each solid support of the library can be greater than or equal to 0.08 mmol per gram. The loading of the PNA dimer on each solid support of the library can be in the range from about 0.1 mmol per gram to about 1 mmol per gram. The loading of the PNA dimer on each solid support of the library can be in the range from about 0.12 mmol per gram to about 0.35 mmol per gram (See Example 1).

The library of supports can be arranged to produce an array.

### d) A Method For Forming PNA Dimer Solid Supports

In some embodiments, this invention pertains to a method for forming a support bound PNA dimer. The method comprises coupling a first PNA monomer to a sterically hindered solid support wherein the PNA monomer comprises a N-terminal amine base labile protecting group. Optionally, but preferably, the solid support is washed to remove excess first PNA monomer. The solid support is then treated for a period of about 1 to about 2 minutes with a deprotection reagent that substantially removes the base labile N-terminal amine protecting group from the support bound first PNA monomer. This deprotection step should be performed quickly because the unprotonated N-terminal amine can attack the acid forming cleavable linker and thereby cause cyclization and elimination of the first PNA monomer from the support. When performed quickly, it is possible to obtain less than 50 percent cyclization and elimination of the first PNA monomer. Once the deprotection is performed, the solid support can be washed to remove the deprotection reagent. Again this washing should be performed quickly. By quickly we mean that it should be performed as quickly as it can reasonably be performed; generally no more than 2-10 minutes elapsing between the time the deprotection reagent is first applied to the solid support and the time the coupling of the second PNA monomer is commenced. After washing, a second PNA monomer is coupled to the N-terminal amine of the first PNA monomer as soon as is practical and preferable within 2-10 minutes of the time the deprotection reagent is first applied to the solid support.

According to this embodiment, the first and second PNA monomer can be a Fmoc(Bhoc) PNA monomer comprising the same or a different nucleobase. The nucleobase of the first and/or second PNA monomer can be independently selected from the group consisting of: adenine, cytosine, guanine, thymine, uracil, 5-propynyl-uracil, 2-thio-5-propynyl-uracil, 5-methylcytosine, pseudoisocytosine, 2-thiouracil and 2-thiothymine, 2-aminopurine, N9-(2-amino-6-chloropurine), N9-(2,6-diaminopurine), hypoxanthine, N9-(7-deaza-guanine), N9-(7-deaza-8-aza-guanine) and N8-(7-deaza-8-aza-adenine).

The N-terminal base labile protecting group of the first or second PNA monomer can be Fmoc. The deprotection reagent can be a solution containing from about 15 to about 25 (v/v) percent piperidine in an organic solvent. Non-limiting examples of suitable organic solvents include N,N'-dimethlyformamide (DMF) and 1-Methyl-2-pyrrolidone (NMP). For example, the deprotection reagent can be 20 percent (v/v) piperidine in N,N'-dimethlyformamide (DMF).

The deprotection reagent can be a solution containing from about 0.2 % to about 4% DBU (v/v) in an organic solvent. For example, the deprotection reagent can be 2% DBU in NMP (v/v).

According to this embodiment, the sterically hindered solid support can be selected from the group consisting of: Trityl chloride resin (Trityl-Cl), 2-Chlorotrityl chloride resin, DHPP, MBHA, 4-methyltrityl chloride resin, 4-methoxytrityl chloride resin, Hydroxy-(2-chorophenyl)methyl-PS, Rink Acid Resin and NovaSyn TGT alcohol resin. Preferably, the sterically hindered solid support is Trityl chloride resin.

### e) Another Method For Forming PNA Dimer Solid Supports

In some embodiments, this invention pertains to a yet another method for forming a support bound PNA dimer. The method comprises coupling a first PNA monomer to solid support comprising an acid forming cleavable linker wherein the PNA monomer comprises an acid labile N-terminal protecting group. Optionally, but preferably, the solid support is washed to remove excess first PNA monomer. The solid support is then treated with a deprotection reagent under acidic conditions that deprotect the acid labile N-terminal protecting group. Once the deprotection is performed, the solid support can be washed to remove the deprotection reagent. After washing, a second PNA monomer is coupled to the N-terminal amine of the first PNA monomer. According to the method, the final loading of the PNA dimer on the solid support is greater than or equal to 0.08 mmol per gram. Unlike the method described above, there is no requirement that the deprotection step be performed quickly as the N-terminal amine becomes protonated and thereby unable to cause cyclization and elimination of the first PNA synthon.

The first and second PNA monomers can be t-boc/Z protected PNA monomers comprising the same or a different nucleobase. The first and second PNA monomers can be Mmt/Bhoc protected PNA monomers comprising the same or a different nucleobase. The first PNA monomer can be an Mmt/Bhoc protected PNA monomer and the second PNA monomer can be an Fmoc/Bhoc protected PNA monomer.

The nucleobase of the first and second PNA monomer can be independently selected from the group consisting of: adenine, cytosine, guanine, thymine, uracil, 5-propynyl-uracil, 2-thio-5-propynyl-uracil, 5-methylcytosine, pseudoisocytosine, 2-thiouracil and 2-thiothymine, 2-aminopurine, N9-(2-amino-6-chloropurine), N9-(2,6-diaminopurine), hypoxanthine, N9-(7-deaza-guanine), N9-(7-deaza-8-aza-guanine) and N8-(7-deaza-8-aza-adenine).

According to the method, where the first PNA monomer is an Mmt/Bhoc protected PNA monomer and the deprotection reagent can be a solution containing from about 1 to about 5 percent (v/v) dichloroacetic acid in an organic solvent. For example, the deprotection reagent can be about 2 percent dichloroacetic acid (DCA) in dichloromethane (DCM).

According to the method, the solid support can be a sterically hindered solid support is selected from the group consisting of: Trityl chloride resin (Trityl-Cl), 2-Chlorotrityl chloride resin, DHPP, MBHA, 4-methyltrityl chloride resin, 4-methoxytrityl chloride resin, Hydroxy-(2-chorophenyl)methyl-PS, Rink Acid Resin and NovaSyn TGT alcohol resin. The solid support can also be selected from the group consisting of: Fmoc-PAL-PEG-PS, NovaSyn TGA and Wang Resin.

According to the method, the final loading of the PNA dimer on the solid support can be in the range from about 0.1 mmol per gram to about 1.2 mmol per gram. The final loading of the PNA dimer on the solid support can in the range from about 0.12 mmol per gram to about 0.35 mmol per gram.

### f) PNA C-Terminal Acid Oligomers

In yet another embodiment, this invention pertains to a PNA C-terminal acid oligomer comprising a C-terminal PNA subunit and a fluorescent label or quencher. For example, the fluorescent label can be Dye 1 or Dye 2. For example, the quencher can be dabcyl. The label can be linked to the N-terminal subunit of the PNA oligomer, including to the N-terminal amine.

The PNA oligomer can be 10 or less PNA subunits in length. Such short oligomers can be conveniently used for the preparation of elongated PNA oligomers and PNA chimeras using ligation/condensation through a library approach. For example, the PNA oligomer can be from about 3 to about 8 subunits in length or from about 4 to about 6 subunits in length. The PNA oligomer can be 4 subunits in length. The PNA oligomer can be 5 subunits in length.

The nucleobases of the PNA oligomer can be selected from the group from the group consisting of: adenine, cytosine, guanine, thymine, uracil, 5-propynyl-uracil, 2-thio-5-propynyl-uracil, 5-methylcytosine, pseudoisocytosine, 2-thiouracil and 2-thiothymine, 2-aminopurine, N9-(2-amino-6-chloropurine), N9-(2,6-diaminopurine), hypoxanthine, N9-(7-deaza-guanine), N9-(7-deaza-8-aza-guanine) and N8-(7-deaza-8-aza-adenine).

### g) Library of PNA C-terminal Acid Oligomers

In still another embodiment, this invention pertains to a library of PNA C-terminal acid oligomers. Each PNA C-terminal acid oligomer of the library comprises a nucleobase sequence, a C-terminal PNA subunit (not an amino acid such as glycine or lysine) and a fluorescent label or quencher moiety. The fluorescent label or quencher moiety of each PNA oligomer can be linked to the N-terminal subunit, including without limitation, to the N-terminal amine. Each PNA oligomer of the library differs, either in label, nucleobase sequence, subunit length or polarity of nucleobase sequence, from each of the other PNA oligomers of the library. The nucleobases of each PNA oligomer of the library can be selected from the group from the group consisting of: adenine, cytosine, guanine, thymine, uracil, 5-propynyl-uracil, 2-thio-5-propynyl-uracil, 5-methylcytosine, pseudoisocytosine, 2-thiouracil and 2-thiothymine, 2-aminopurine, N9-(2-amino-6-chloropurine), N9-(2,6-diaminopurine), hypoxanthine, N9-(7-deaza-guanine), N9-(7-deaza-8-aza-guanine) and N8-(7-deaza-8-aza-adenine).

Each PNA oligomer of the library can comprise the same number of PNA subunits, provided however that this is not a limitation. PNA oligomers of a library can comprise a different number of PNA subunits. For example, the library can comprise at least one PNA oligomer that has a different number of PNA subunits as compared to at least one other PNA oligomer of the library. Each PNA oligomer of the library can comprise from about 3 to about 8 PNA subunits. Each PNA oligomer of the library can comprise from about 4 to about 6 PNA subunits. Each PNA oligomer of the library can comprise 5 PNA subunits.

A library of PNA C-terminal acid oligomers can itself be a set within a larger library. For example a library can comprise three or more sets of oligomer blocks wherein at least two sets can be substantially identical except for the nature of the label such that the two or more different labels renders each set of oligomer blocks independently detectable. The two or more independently detectable oligomer block sets can be sets of terminal oligomer blocks or condensation oligomer blocks, including for example two sets PNA C-terminal acids wherein each set is labeled with a different fluorophore (e.g. Dye 1 and Dye2). By producing two sets of oligomer blocks that are essentially identical but for the nature of the attached independently detectable label, it is possible to prepare pairs of independently detectable elongated oligomers, including self-indicating independently detectable oligomer, through ligation/condensation with a common oligomer block. By self-indicating we mean that the probes change detectable properties upon hybridization to a target sequence and thereby reduce or eliminate the requirement for the removal of excess probe. By independently detectable we mean that it is possible to determine one label independently, and optionally in the presence of, the other label.

For example, at least two oligomer blocks comprising independently detectable labels can be ligated to the same oligomer blocks that, for example, is labeled with a quencher moiety. The pair of independently detectable self-indicating elongated oligomers can then be used as probes for performing SNP genotyping assays as described in United States published application 2003-0077608 and PCT published application WO02/072865, provided however that the PNA oligomers produced by this reaction can be native PNA oligomers.

If an acceptor or quencher moiety is not present on one of the oligomer blocks that are ligated/condensed, then the elongated oligomers might be an oligomer with a single label. If a label is not present on either of the oligomer blocks that are ligated/condensed, then the elongated oligomers might be an unlabeled oligomer that can, for example, be used as blocking probes (See for Example: US 6,110,676) or used as a capture probe.

**TABLE 1; Configuration Of Potential Oligomer Block Sets Of A Library**

| **Condensation Block Set** | **Terminal Block Set** | **Properties of Elongated Oligomer/Potential Applications** |
|---|---|---|
| Unlabeled | Unlabeled | Unlabeled probe or primer; blocking probe, capture probe or detector probe |
| Unlabeled | Label | Labeled probe or primer |
| Label | Unlabeled | Labeled probe or primer |
| Donor/Acceptor | Donor/Acceptor | Self-Indicating Probe |
| Donor/Acceptor | Unlabeled | Component Polymer of Detection Complex |
| Unlabeled | Donor/Acceptor | Component Polymer of Detection Complex |

In accordance with the prior description, Table 1 summarizes various possibilities for the make up of sets of oligomer blocks of a possible library as well as the properties of the elongated oligomers prepared by the ligation thereof. Of course Table 1 is not intended to be exhaustive of possibilities. Moreover, the PNA C-terminal acid oligomers can themselves one or more sets or subsets of blocks of a larger library of PNA oligomers (such as summarized in Table 1) that can, *inter alia,* be used for the preparation of PNA oligomers or PNA chimeras.

One or more of the sets or subsets of oligomer blocks can also optionally contain protected or unprotected functional groups linked to the oligomer blocks at the termini or linked at a position internal to the oligomer blocks. In this regard, the oligomer blocks can be labeled either pre- or post-ligation, depending on a practitioner's desire and available resources. The functional groups can also be used to attach the oligomer blocks or formed elongated oligomers to a surface.

Accordingly, in another embodiment, this invention pertains to a library that comprises at least two sets of PNA oligomers wherein the PNA oligomers of each set differ from those of the other set primarily in the nature of the fluorescent label. Such PNA C-terminal acid oligomers can be used in ligation/condensation reactions to produce probes or sets of probes for SNP genotyping assays as described in United States published application 2003-0077608 and PCT published application WO02/072865, provided however that the PNA oligomers produced by this reaction can be native PNA oligomers.

The invention also pertains to the following:
1. A solid support composition comprising:
   a) an acid forming cleavable linker; and
   b) a PNA dimer, comprising an N-terminal base labile protecting group, cleavably linked to the solid support through the cleavable linker, wherein the loading of the PNA dimer on the solid support is greater than or equal to 0.08 mmol per gram.
2. The composition of item 1, wherein the solid support is a sterically hindered solid support.
3. The composition of item 2, wherein the sterically hindered solid support is selected from the group consisting of: Trityl chloride resin (Trityl-Cl), 2-Chlorotrityl chloride resin, DHPP, MBHA, 4-methyltrityl chloride resin, 4-methoxytrityl chloride resin,
   Hydroxy-(2-chorophenyl)methyl-PS, Rink Acid Resin and NovaSyn TGT alcohol resin.
4. The composition of item 1, wherein the solid support is selected from the group consisting of: PAL-PEG-PS™, NovaSyn TGA and Wang Resin.
5. The composition of item 1 or 2, wherein the PNA dimer is linked to the cleavable linker by an ester bond.
6. The composition of item 1 or 2, wherein the PNA dimer is formed from Fmoc(Bhoc) monomers.
7. The composition of item 1 or 2, wherein the loading of the PNA dimer on the solid support is in the range from about 0.1 mmol per gram to about 1 mmol per gram.
8. The composition of item 1 or 2, wherein the loading of the PNA dimer on the solid support is in the range from about 0.12 mmol per gram to about 0.35 mmol per gram.
9. The composition of item 1 or 2 wherein the solid support is an array comprising two or more different support bound PNA dimers.
10. A library comprising at least two solid supports wherein said at least two solid supports each comprise:
   a) an acid forming cleavable linker; and
   b) a PNA dimer that: (i) is cleavably linked to the acid forming cleavable linker; and (ii) differs in nucleobase sequence from the PNA dimer that is linked to any of the other of the at least two solid supports of the library.
11. The library of item 10, wherein the library comprises at least sixteen solid supports, each support comprising a PNA dimer chosen from a set of at least sixteen possible PNA dimers wherein each PNA dimer of the set differs from all of the other PNA dimers of the set by at least one of at least four different nucleobases.
12. The library of item 11, wherein each of the at least four different nucleobases is selected from the group consisting of: adenine, cytosine, guanine, thymine, uracil, 5-propynyl-uracil, 2-thio-5-propynyl-uracil, 5-methylcytosine, pseudoisocytosine, 2-thiouracil and 2-thiothymine, 2-aminopurine, N9-(2-amino-6-chloropurine), N9-(2,6-diaminopurine), hypoxanthine, N9-(7-deaza-guanine), N9-(7-deaza-8-aza-guanine) and N8-(7-deaza-8-aza-adenine).
13. The library of item 10, wherein the solid support is a sterically hindered solid support.
14. The library of item 13, wherein the sterically hindered solid support is selected from the group consisting of: Trityl chloride resin (Trityl-Cl), 2-Chlorotrityl chloride resin, DHPP, MBHA, 4-methyltrityl chloride resin, 4-methoxytrityl chloride resin, Hydroxy-(2-chorophenyl)methyl-PS, Rink Acid Resin and NovaSyn TGT alcohol resin.
15. The library of item 10, wherein the solid support is selected from the group consisting of: PAL-PEG-PS, NovaSyn TGA and Wang Resin.
16. The library of item 10 or 13, wherein the PNA dimer is linked to the cleavable linker by an ester bond.
17. The library of item 16, wherein the C-terminal subunit of the PNA dimer is linked to the cleavable linker.
18. The library of item 13, wherein the PNA dimer is formed from Fmoc(Bhoc) protected PNA monomers.
19. The library of item 10 or 13, wherein the PNA dimer is formed from t-boc/Z protected PNA monomers.
20. The library of item 10 or 13, wherein the PNA dimer is formed from Mmt/Bhoc protected PNA monomers.
21. The library of item 10 or 13, wherein the PNA dimer is formed from both Mmt/Bhoc protected PNA monomers and Fmoc(Bhoc)protected PNA monomers.
22. The library of item 10 or 13, wherein the loading of the PNA dimer on at least one solid support of the library is greater than or equal to 0.08 mmol per gram.
23. The library of item 10 or 13, wherein the loading of the PNA dimer on at least one half of the solid supports of the library is greater than or equal to 0.08 mmol per gram.
24. The library of item 10 or 13, wherein the loading of the PNA dimer on all of the solid supports of the library is greater than or equal to 0.08 mmol per gram.
25. The library of item 24, wherein the loading of the PNA dimer on each solid support of the library is in the range from about 0.1 mmol per gram to about 1 mmol per gram.
26. The library of item 24, wherein the loading of the PNA dimer on each solid support of the library is in the range from about 0.12 mmol per gram to about 0.35 mmol per gram.
27. The library of item 10 or 13, wherein the library of supports is arranged to produce an array.
28. A method for forming a support bound PNA dimer, said method comprising:
   a) coupling a first PNA monomer to a sterically hindered solid support comprising a sterically hindered acid forming cleavable linker wherein the PNA monomer comprises a N-terminal amine base labile protecting group;
   b) optionally washing the solid support to remove excess first PNA monomer;
   c) treating the solid support for a period of about 1 to about 2 minutes with a deprotection reagent that substantially removes the base labile N-terminal amine protecting group from the support bound first PNA monomer but that does not allow for more than 50 percent cyclization and elimination of the first PNA monomer from the support;
   d) washing the solid support to remove the deprotection reagent; and
   e) coupling a second PNA monomer to the N-terminal amine of the first PNA monomer as soon as is practical after performing steps (c) and (d).
29. The method of item 28, wherein the first and second PNA monomers are Fmoc(Bhoc) PNA monomers comprising the same or a different nucleobase.
30. The method of item 29, wherein the nucleobase of the first and second PNA monomer is independently selected from the group consisting of: adenine, cytosine, guanine, thymine, uracil, 5-propynyl-uracil, 2-thio-5-propynyl-uracil, 5-methylcytosine, pseudoisocytosine, 2-thiouracil and 2-thiothymine, 2-aminopurine, N9-(2-amino-6-chloropurine), N9-(2,6-diaminopurine), hypoxanthine, N9-(7-deaza-guanine), N9-(7-deaza-8-aza-guanine) and N8-(7-deaza-8-aza-adenine).
31. The method of item 28, wherein the N-terminal base labile protecting group is Fmoc.
32. The method of item 28, wherein the deprotection reagent is a solution containing from about 15 to about 25 percent piperidine in an organic solvent.
33. The method of item 32, wherein the deprotection reagent is 20 percent piperidine in N,N'-dimethlyformamide (DMF).
34. The method of item 28, wherein the deprotection reagent is a solution containing from about 0.2% to about 4% (v/v) DBU in NMP.
35. The method of item 34, wherein the deprotection reagent is about 2% DBU in NMP.
36. The method of item 28, wherein the sterically hindered solid support is selected from the group consisting of: Trityl chloride resin (Trityl-Cl), 2-Chlorotrityl chloride resin, DHPP, MBHA, 4-methyltrityl chloride resin, 4-methoxytrityl chloride resin, Hydroxy-(2-chorophenyl)methyl-PS, Rink Acid Resin and NovaSyn TGT alcohol resin.
37. The method of item 28, wherein the sterically hindered solid support is Trityl chloride (Trityl-Cl) resin.
38. The method of item 28, wherein the final loading of the PNA dimer on the solid support is greater than or equal to 0.08 mmol per gram.
39. The method of item 28, wherein the final loading of the PNA dimer on the solid support is in the range from about 0.1 mmol per gram to about 1 mmol per gram.
40. The method of item 28, wherein the final loading of the PNA dimer on the solid support is in the range from about 0.12 mmol per gram to about 0.35 mmol per gram.
41. A method for forming a support bound PNA dimer, said method comprising:
   a) coupling a first PNA monomer to solid support comprising an acid forming cleavable linker wherein the PNA monomer comprises an acid labile N-terminal protecting group;
   b) optionally washing the solid support to remove excess first PNA monomer;
   c) treating the solid support with a deprotection reagent under acidic conditions that deprotect the acid labile N-terminal protecting group;
   d) washing the solid support to remove the deprotection reagent; and
   e) coupling a second PNA monomer to the N-terminal amine of the first PNA monomer,
   wherein the final loading of the PNA dimer on the solid support is greater than or equal to 0.08 mmol per gram.
42. The method of item 41, wherein the first and second PNA monomers are t-boc/Z protected PNA monomers comprising the same or a different nucleobase.
43. The method of item 41, wherein the first and second PNA monomers are Mmt/Bhoc protected PNA monomers comprising the same or a different nucleobase.
44. The method of item 41, wherein the first PNA monomer is an Mmt/Bhoc protected PNA monomer and the second PNA monomer is an Fmoc/Bhoc protected PNA monomer.
45. The method of item 41, wherein the nucleobase of the first and second PNA monomer is independently selected from the group consisting of: adenine, cytosine, guanine, thymine, uracil, 5-propynyl-uracil, 2-thio-5-propynyl-uracil, 5-methylcytosine, pseudoisocytosine, 2-thiouracil and 2-thiothymine, 2-aminopurine, N9-(2-amino-6-chloropurine), N9-(2,6-diaminopurine), hypoxanthine, N9-(7-deaza-guanine), N9-(7-deaza-8-aza-guanine) and N8-(7-deaza-8-aza-adenine).
46. The method of item 41, wherein the first PNA monomer is an Mmt/Bhoc protected PNA monomer and the deprotection reagent is a solution containing from about 1 to about 5 percent (v/v) dicloroacetic acid in an organic solvent.
47. The method of item 46, wherein the deprotection reagent is about 2 percent dichloroacetic acid in dichloromethane (DCM).
48. The method of item 41, wherein the solid support is a sterically hindered solid support is selected from the group consisting of: Trityl chloride resin (Trityl-Cl), 2-Chlorotrityl chloride resin, DHPP, MBHA, 4-methyltrityl chloride resin, 4-methoxytrityl chloride resin, Hydroxy-(2-chorophenyl)methyl-PS, Rink Acid Resin and NovaSyn TGT alcohol resin.
49. The method of item 41, wherein the solid support is selected from the group consisting of: Fmoc-PAL-PEG-PS, NovaSyn TGA and Wang Resin.
50. The method of item 41, wherein the final loading of the PNA dimer on the solid support is in the range from about 0.1 mmol per gram to about 1.2 mmol per gram.
51. The method of item 41, wherein the final loading of the PNA dimer on the solid support is in the range from about 0.12 mmol per gram to about 0.35 mmol per gram.
52. A PNA C-terminal acid oligomer comprising a C-terminal PNA subunit and a fluorescent label or quencher.
53. The PNA oligomer of item 52, wherein the fluorescent label is Dye 1 or Dye 2.
54. The PNA oligomer of item 52, wherein the quencher moiety is dabcyl.
55. The PNA oligomer of item 52, wherein the PNA oligomer is 10 or less PNA subunits in length.
56. The PNA oligomer of item 52, wherein the PNA oligomer is from about 3 to about 8 subunits in length.
57. The PNA oligomer of item 52, wherein the oligomer is from about 4 to about 6 subunits in length.
58. The PNA oligomer of item 52, wherein the PNA oligomer is 4 subunits in length.
59. The PNA oligomer of item 52, wherein the PNA oligomer is 5 subunits in length.
60. The PNA oligomer of item 52, wherein the label is linked to the N-terminal subunit of the PNA oligomer.
61. The PNA oligomer of item 52, wherein the label is linked to the N-terminal amine of the PNA oligomer.
62. The PNA oligomer of item 52, wherein the nucleobases of the oligomer are selected from the group from the group consisting of: adenine, cytosine, guanine, thymine, uracil, 5-propynyl-uracil, 2-thio-5-propynyl-uracil, 5-methylcytosine, pseudoisocytosine, 2-thiouracil and 2-thiothymine, 2-aminopurine, N9-(2-amino-6-chloropurine), N9-(2,6-diaminopurine), hypoxanthine, N9-(7-deaza-guanine), N9-(7-deaza-8-aza-guanine) and N8-(7-deaza-8-aza-adenine).
63. A library of PNA C-terminal acid oligomers, each PNA oligomer of the library comprising:
   a) a nucleobase sequence;
   b) a C-terminal PNA subunit; and
   c) a fluorescent label or quencher moiety;
   wherein each PNA oligomer differs, either in label, nucleobase sequence, subunit length or polarity of nucleobase sequence, from each of the other PNA oligomers of the library.
64. The library of item 63, wherein the nucleobases of each PNA oligomer are selected from the group from the group consisting of: adenine, cytosine, guanine, thymine, uracil, 5-propynyl-uracil, 2-thio-5-propynyl-uracil, 5-methylcytosine, pseudoisocytosine, 2-thiouracil and 2-thiothymine, 2-aminopurine, N9-(2-amino-6-chloropurine), N9-(2,6-diaminopurine), hypoxanthine, N9-(7-deaza-guanine), N9-(7-deaza-8-aza-guanine) and N8-(7-deaza-8-aza-adenine).
65. The library of item 63, wherein the fluorescent label or quencher of each PNA oligomer is linked to the N-terminal subunit.
66. The library of item 63, wherein the fluorescent label or quencher of each PNA oligomer is linked to the N-terminal amine.
67. The library of item 63, wherein each PNA oligomer of the library comprises the same number of PNA subunits.
68. The library of item 63, wherein at least one of the PNA oligomers of the library comprise a different number of PNA subunits as compared to at least one other PNA oligomer of the library.
69. The library of item 63, wherein each PNA oligomer of the library comprises from about 3 to about 8 PNA subunits.
70. The library of item 63, wherein each PNA oligomer of the library comprises from about 4 to about 6 PNA subunits.
71. The library of item 63, wherein each PNA oligomer of the library comprises 4 PNA subunits.
72. The library of item 63, wherein each PNA oligomer of the library comprises 5 PNA subunits.
73. The library of item 63, wherein the library comprises at least two sets of PNA C-terminal acid oligomers wherein the PNA oligomers of each set differ from those of the other set primarily in the nature of a fluorescent label.
74. The library of item 73, wherein the first set of PNA C-terminal acid oligomers is labeled with Dye1 and the second set of PNA C-terminal acid oligomers is labeled with Dye2.

### Brief Description Of The Drawings:

Figure 1 is the result of a high performance liquid chromatography (HPLC) analysis of a sample of crude fluorescently labeled PNA C-terminal acid pentamer.
Figure 2 is a schematic illustration of a N-terminal amine protecting group shuffle reaction for producing Mmt/Bhoc monomers from Fmoc(Bhoc) PNA monomers.
Figure 3 is a schematic illustration of N-terminal amine protecting group removal and inhibition of ketopiperazine formation (cyclization and elimination) by N-terminal amine group protonation.
Figure 4 is a schematic of the preparation of a fluorescently labeled PNA C-terminal acid oligomer starting from Wang resin (solid support) and a combination of Mmt/Bhoc and Fmoc(Bhoc) PNA monomers.
Figure 5 is the structure of bis-(t-boc)-protected Dye1.
Figure 6 is an illustration of NovaSyn-TGA Resin.
Figure 7 is the structure of Dye1 and Dye2.
Figures 8a and 8b are illustrations of non-limiting examples of PNA ligation/condensation reactions that can be used to produce elongated PNA oligomers and PNA chimeras.

Having described the forgoing embodiments of the invention, the following examples are intended to be illustrative but not intended to be limiting in any way.

### Modes For Carrying Out The Invention:

This invention is now illustrated by the following examples that are not intended to be limiting in any way.

### Example 1; Synthesis Of A Library Of PNA Dimer-Resins (Solid Supports)

### I. Loading of first PNA-monomers on Trityl-Cl resin

*Reagents:* Fmoc(Bhoc)-PNA monomers (Fmoc-A(Bhoc)-OH: P/N GEN063014, Fmoc-C(Bhoc)-OH: P/N GEN063015, Fmoc-G(Bhoc)-OH: P/N GEN063016, Fmoc-T-OH: P/N GEN063017, 1-Methyl-2-pyrrolidone (NMP): P/N 400580, N,N-Dimethylformamide (DMF): P/N 400143, N,N-Diisopropylethylamine (DIPEA): P/N GEN0750000 and O-(7-Azabenzotriazol-1-yl)-N,N,N',N'-tetramethyluronium hexafluorophosphate (HATU): P/N GEN063080 were all obtained from Applied Biosystems, Foster City, CA. Methanol: P/N 015-4 and acetonitrile: P/N 230-4 were obtained from Burdick & Jackson (Muskegon, Michigan). Trityl-Cl resin: P/N 01-64-0074 was obtained from Novabiochem (San Diego, CA) in 5 g batches. Dry solvents were used for trityl-chloride resin loading reactions. Anhydrous dichloromethane (CH₂Cl₂): P/N 27,099-7, NMP was stored over 4Å molecular sieves overnight. Solvents (DMF, NMP and acetonitrile) used for resin washing and other reactions were of reagent grade.

*Procedure:* Fmoc-A(Bhoc)-OH, Fmoc-G(Bhoc)-OH and Fmoc-T-OH monomers (3.62, 3.69 and 2.53 g respectively, 4.98 mmol) and 2.63 mL of DIPEA (∼3 x 4.98 mmol) were dissolved in 15 mL of dry 4:1 CH₂Cl₂-NMP (0.332 M). Fmoc-C(Bhoc)-OH monomer (3.49 g, 4.98 mmol) and 2.63 mL of DIPEA (∼3 x 4.98 mmol) was dissolved first in 12 mL of NMP and then the volume was adjusted to 15 mL by the addition of dry CH₂Cl₂.

Monomer solutions were then added to dry Trityl-Cl resin (3g/reaction, 1.66 mmol/g) in 50 mL plastic tubes, capped and shaken for 0.5 hour at room temperature when the resin became a viscous gel. At this point another 3 mL of dry CH₂Cl₂ was added to each reaction mixture and the reaction continued for another 2.5 h. (A recent study showed that the reaction is essentially complete within 45 minute and the loading capacity of the resin does not increase anymore by prolonging the reaction time.) The resin was then filtered and washed twice with NMP (∼15 mL each), followed by a quick wash with 15 mL of CH₂Cl₂-MeOH-DIPEA (17:2:1, v/v) and a couple of washes with NMP. The final wash was performed with acetonitrile and the resin was then dried under vacuum.

Resin loading was calculated by swelling the resin with THF for at least 2 hour before the piperidine treatment (for 1 h) while conducting an Fmoc count experiment. In a typical Fmoc count experiment, 3-5 mg of the dimer-resin was treated with 100 µL of THF in a capped 1 mL microcentrifuge tube for 2 h followed by the addition of 200 µL of 20% piperidine in DMF (v/v). The reaction mixture was then vortexed and allowed to stand for 1 hour. The volume of the reaction mixture was adjusted to 1 mL by addition of methanol and mixed thoroughly. The supernatant was diluted 20 times with methanol and UV absorbance was recorded at 301 nm (background correction was done with methanol). Molar extinction coefficient (ε = 7800 M⁻¹Cm⁻¹ at 301 nm) of the chromophore released upon Fmoc cleavage was used to calculate the loading capacity of the resin. The results of the loading determination are found in Table 2. Note: PNA monomer resins should be used within a day or two of synthesis and be stored at 4°C until used.

**Table 2. Loading capacity of the monomer-trityl resins.**

| *Resin* | **Loading (mmole/g)** |
|---|---|
| Fmoc-A^{Bhoc}-Trityl | 0.39 |
| Fmoc-C^{Bhoc}-Trityl | 0.81 |
| Fmoc-G^{Bhoc}-Trityl | 0.68 |
| Fmoc-T-Trityl | 0.48 |

### II. Step B: Coupling of the second monomer

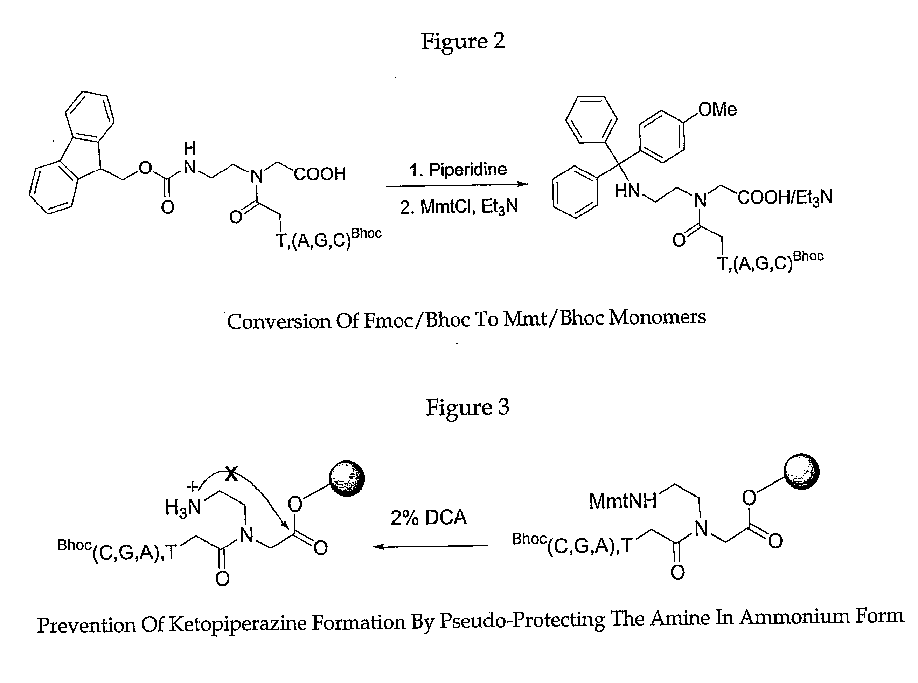

About 0.5 g of each dry resin obtained from Experiment I above was swelled with NMP for at least 2-3 hour. The Fmoc group was then removed by treating the resin for 1 minute with 5 mL of 20% piperidine in DMF (v/v). While the Fmoc deprotection was in progress a solution of PNA monomer (4 equiv) in NMP was activated by the addition of DIPEA (9 equiv) and HATU (3.8 equiv). Final monomer concentration was 0.17 M. The resin was then washed with NMP (3 x 10-15 mL) within another minute, followed by the addition pf the activated PNA-monomer. The coupling was performed for 15 minutes and the resin was then washed with NMP and acetonitrile before drying under vacuum. Resin loading capacity was then calculated by Fmoc determination. All possible 16 dimer-resins (based on the 4 nucleobases A, C, G & T) were synthesized using the above protocol. Loading capacity (Table 3) of PNA dimer-resins was in the range of 0.12-0.35 mmole/g. This loading is often used in conventional Fmoc-XAL-PEGPS resin. Note: Whenever: 1) the cleavable linker forms a C-terminal acid; and 2) the deprotection of the N-α-amine is performed under basic conditions, the coupling reaction of the second PNA oligomer should be performed as soon as is practical after the removal of the N-terminal protecting group since a cyclization and elimination reaction (see figure 3) can occur whereby a substantial portion of first monomer is removed from the solid support to thereby effectively lower the loading of the resin.

The dimer-resins that were prepared appear to be stable when stored like any other conventional trityl resins (4 °C). Loading capacity of the resin stored under such conditions remained constant for at least four months. To test the stability of dimer-resin, a sample of the resin, which was stored at 4 °C for four months, was washed with DMF and dried before checking the loading capacity by Fmoc determination (see the procedure set forth above). The washings were free of any UV active compound thereby indicating that they do not substantially degrade under these conditions.

**Table 3. Loading capacity of the dimer-trityl-Cl resins.**

| Resin | Loading (mmole/g) |
|---|---|
| Fmoc-A^{Bhoc}G^{Bhoc}-Trityl | 0.17 |
| Fmoc-C^{Bhoc}G^{Bhoc}-Trityl | 0.29 |
| Fmoc-G^{Bhoc}G^{Bhoc}-Trityl | 0.30 |
| Fmoc-TG^{Bhoc}-Trityl | 0.23 |
| Fmoc-A^{Bhoc}C^{Bhoc}-Trityl | 0.18 |
| Fmoc-C^{Bhoc}C^{Bhoc}-Trityl | 0.31 |
| Fmoc-G^{Bhoc}C^{Bhoc}-Trityl | 0.29 |
| Fmoc-TC^{Bhoc}-Trityl | 0.24 |
| Fmoc-A^{Bhoc}A^{Bhoc}-Trityl | 0.16 |
| Fmoc-C^{Bhoc}A^{Bhoc}-Trityl | 0.28 |
| Fmoc-G^{Bhoc}A^{Bhoc}-Trityl | 0.19 |
| Fmoc-TA^{Bhoc}-Trityl | 0.21 |
| Fmoc-A^{Bhoc}T-Trityl | 0.12 |
| Fmoc-C^{Bhoc}T-Trityl | 0.34 |
| Fmoc-G^{Bhoc}T-Trityl | 0.35 |
| Fmoc-TT-Trityl | 0.24 |

### Example 2: Synthesis Of PNA C-Terminal Acid Oligomers Using Dimer-Resin

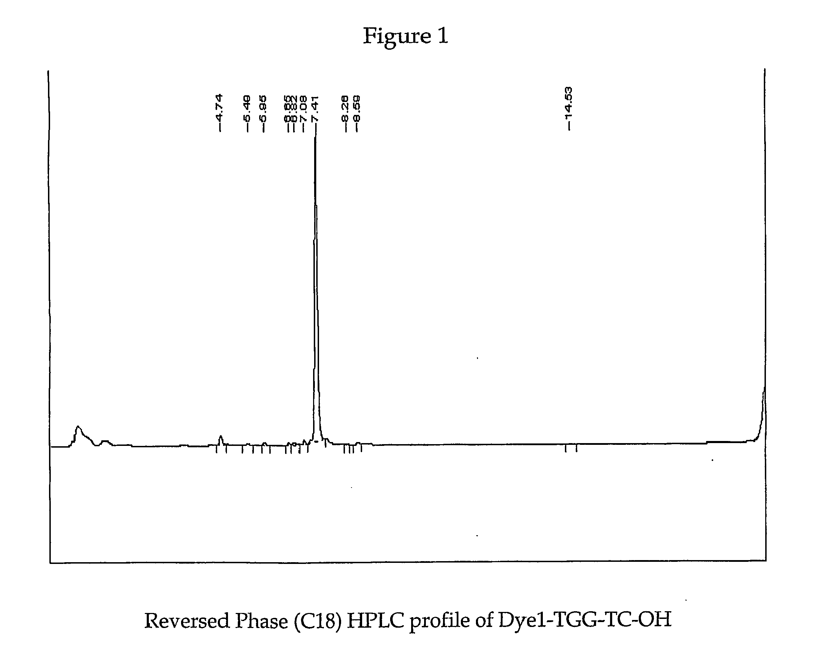

Table 4 identifies and provides synthesis data for four PNA pentamers that were among the first PNA oligomers to be assembled using the dimer-resins prepared as described above. Synthesis was performed on an Expedite PNA synthesizer. Dye1 and Dye2 labeled PNA pentamers have now been assembled using the dimer-resins on a regular basis. Figure 1 shows a representative analytical RP-HPLC profile of the crude sample of the PNA oligomer, Dye1-TGG-TC-OH, obtained from such a synthesis.

**Table 4. Pentameric PNA acids.**

| **PNA** | **ε (M⁻¹cm⁻¹)** | **OD** | **Conc.(1mL)** | **% Yield (2µmole)** | **Mass (Calcd)** | **Mass (Obs)** |
|---|---|---|---|---|---|---|
| H-TGC-CC-OH | 40100 | 52.4 | 1.3 mM | 65 | 1328.29 | 1329.73 (MH⁺) |
| H-TGC-CG-OH | 45200 | 43.5 | 0.962 mM | 48 | 1368.31 | 1369.70 (MH⁺) |
| H-TGC-CT-OH | 42100 | 54.1 | 1.28 mM | 64 | 1343.3 | 1345.14 (MH⁺) |
| H-TGC-CA-OH | 47200 | 65.9 | 1.4 mM | 70 | 1352.31 | 1353.83 (MH⁺) |

### Example 3: Synthesis Of PNA Acids Via Monomethoxy trityl (Mmt)/Bhoc Monomers

### I. Preparing The Monomethoxy trityl (Mmt)/Bhoc Monomers

Commercially available Fmoc(Bhoc) PNA monomers were converted to Mmt/Bhoc monomers by shuffling the N-terminal amine protecting group using the reaction as shown in Figure 2. Reactions were simple and yields for all four nucleobases (A/T/G/C) were satisfactory (68-96%).

To perform the N-terminal amine protecting group shuffle, the following additional reagents were used (Except where noted reagents previously identified were used). Hexanes: P/N AH216-4 was obtained from Burdick & Jackson. Piperidine: P/N 10,409-4, 4-Methoxytrityl chloride (Mmt-Cl): P/N 12920-8 and Ninhydrin: P/N 60-127 were obtained from Aldrich chemical company. Ethyl acetate (EtOAc) was obtained from Mallinckrodt (Paris, Kentucky).

Generally the Fmoc(Bhoc)-PNA monomers (1.38 mmol) were dissolved in 42 mL of DCM-DMF (1:1, v/v) followed by the addition of piperidine (818 µL, 8.28 mmol). After 8 minutes, triethylamine (Et₃N; 2 mL) and Mmt-Cl (1.276 g, 4.14 mmol) was added to the reaction mixture and stirred for 18 h at room temperature. If thin layer chromatography (TLC, Silica plate, 9:1 CH₂Cl₂-CH₃OH + 7 drops of DIPEA/10 mL) showed the presence of free primary-amine (Ninhydrin active, base line spot), another batch of Mmt-Cl (3g, 9.73 mmol) and Et₃N (2 mL) was added and stirred for another 3 h. Generally subsequent TLC showed complete consumption of the primary amine. The volatiles were then removed by rotary-evaporation and the yellow foam was dissolved in minimum volume of CH₂Cl₂. The solution so obtained was then loaded on a pad of silica gel (2 (length) x 5 (diameter) inch, packed with 3:2 EtOAc-hexanes + 0.2% of Et₃N) and first washed with 3:2 EtOAc-hexanes (900 mL) followed by elution with 20% CH₃OH in CH₂Cl₂ + 0.2% Et₃N (600 mL). Upon evaporation of solvent pure product was obtained in 68-96% yield.

### II. Analysis Of Side Reactions

It was theorized that the Mmt group could be removed with a solution of 2% dichloroacetic acid (DCA) in dichloromethane after anchoring the first monomer on the resin. This deprotection strategy was intended to generate the protonated amine group, which should not undergo the cyclization and elimination reaction to form the ketopiperazine (Figure 3). Since the Bhoc group is known to be labile under Mmt group removal conditions, there was a possibility for a branching reaction to occur if free exocyclic amines of the nucleobases were generated under these Mmt deprotection conditions.

To test for this possible side reaction, a model experiment was conducted in which Fmoc(Bhoc) cytosine monomer was anchored to the XAL-PEG-PS resin using standard coupling conditions and then treated with DCA solution for 3 minutes. The resin was then washed and the synthesis continued in a commercially available Expedite nucleic acid synthesizer (using the commercially available Fmoc(Bhoc) monomers and protocols) to assemble a PNA oligomer of sequence GATC. The PNA was then cleaved from resin using standard procedures and the crude oligomer was analyzed by MALDI-TOF MS.

The mass data indicated the presence of the product GATC and the acetylated product GATC^{Ac}. This result was anticipated because the Bhoc deprotected cytosine was acetylated in the subsequent capping step(s). However no branched oligomer was detected in the mass analysis. This suggests that the exocyclic amine of cytosine is not nucleophilic enough to be acylated with PNA monomer under typical HATU coupling. The tetramer was then conveniently converted to the non-acylated oligomer by treatment with 28% aqueous NH₃. This was confirmed by mass analysis in a mass spectrometer. From these results it was concluded that Mmt/Bhoc monomers could be used to synthesize the PNA oligomers comprising a C-terminal acid without worry of oligomer branching.

### III. Preparation of PNA C-terminal Acid Oligomers Using Mmt/Bhoc Monomers

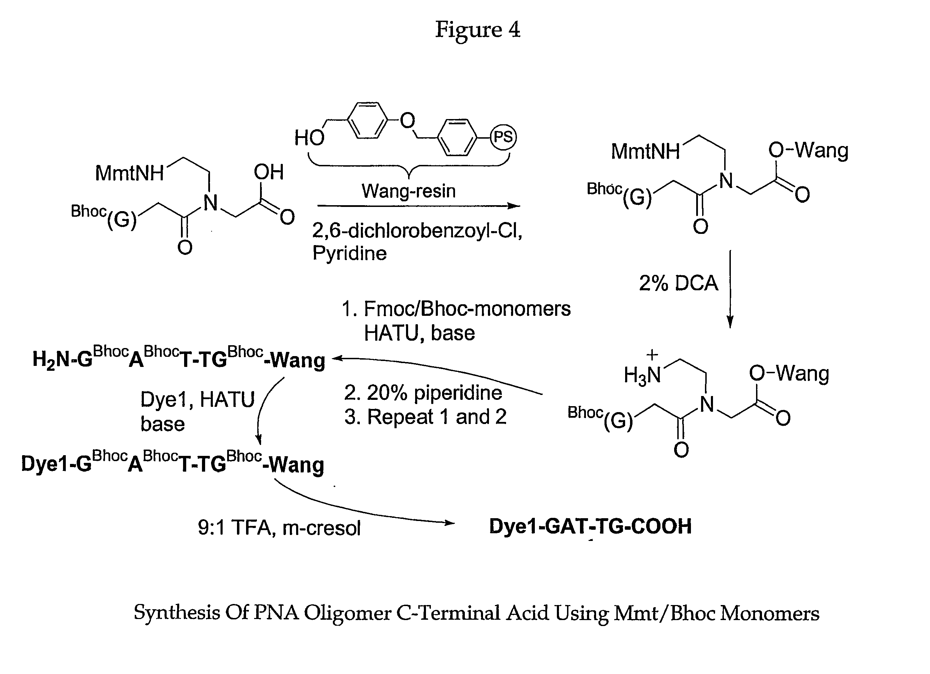

After this determination was made, Mmt/Bhoc-G monomer was anchored to Wang resin using 2,6-dichloro-benzoyl chloride as illustrated in Figure 4. The Mmt group was cleaved using a 2% solution of DCA in dichloromethane. The resin was washed and the next monomer (T, T, G and A) was coupled using standard HATU coupling conditions. No PNA monomers were detected in the DCA washings (TCL: UV and ninhydrin test); which indicated that the first residue is not cleaved from the solid support upon DCA treatment.

The loading was high (0.31 mmol/g; obtained from Fmoc count of the first T) and remained same throughout other couplings. To finish the synthesis, Boc-protected Dye1 (Figure 5) was conjugated at the N-termini of the PNA oligomer using standard labeling conditions. The resin was cleaved and the crude product was analyzed by MALDI-TOF MS and HPLC. The analysis indicated an efficient formation of the product (96%). It is important to note that the capping step was eliminated from all couplings and seems not to affect the quality of the product. This process was also successful using NovaSyn-TGA resin (Figure 6)
wherein resin differs from Wang resin primarily in the presence of a PEG linker. This process can be used to produce a library of PNA dimer resins (solid supports) as described above in Example 1.

### IV. Summary of Results

- Mmt/Bhoc monomer loading on resin is high (1.20-0.27 mmol/g) and can be used for the preparation of PNA C-terminal acid oligomers.
- After the cleavage of Mmt group of the monomer resin, the protonated amine need not be coupled immediately.
- Capping steps can be eliminated with these monomer to reduce time.
- There is no need to change the synthetic protocol of a conventional PNA synthesizer.

The structures of unprotected Dye1 and Dye 2 can be found in Figure 7.

### Example 4: Another Generic Procedure For Dimer Resin Production

*The procedure for monomer resin's synthesis (1-5g scale):*
1) Agitate^{*} Fmoc/Bhoc PNA monomer (1.46 mmol, 0.75 eq.) and DIPEA (4.38 mmol, 2.25 eq.) in NMP/DCM (10mL/5mL, all anhydrous) till the monomer dissolves. Monomer concentration is 0.1M.
2) Add resin^{**} (1.5g, 1.95mmol, 1.00 eq) to the monomer solution; agitate for 4 hrs under inert atmosphere.
3) Filter the resin, wash with 3x15mL NMP with thorough mixing.
4) Agitate the resin in capping solution (DCM/MeOH/DIPEA=17mL/2mL/1mL) for 15 min.
5) Filter the resin, wash with 3x15mL of NMP, 3x15mL of ACN, each with thorough mixing.
6) Dry resin under vacuum.

*The procedure for dimer synthesis (1-5g scale):*
1) Soak monomer resin (0.49 mmol, 1.0 g, 1.00 eq) in 5.0mL of NMP for 3 hrs.
2) Prepare DeFMOC Solution: 2.0% DBU in NMP (5.0mL).
3) Prepare Coupling Solution by agitating the monomer (0.98mmol, 2.00 eq.), HATU (0.93mmol, 1.90eq), and DIPEA (1.96mmol, 4.0eq.) in 10 mL of NMP for 5-10 min. Monomer concentration is 0.1M.
4) Add DeFMOC Solution directly to the monomer resin in NMP, the final concentration of DBU being 1.0%; agitate for 4 min.
5) Filter the beads; wash with 3x15 mL NMP with mixing (takes approx 4 minutes).
6) Add Coupling Solution to the resin and agitate for 60 min.
7) Filter the resin, wash with 3x15mL NMP with thorough mixing.
8) Add 15 mL of PNA Capping Solution (Applied Biosystems, P/N GEN063102) to the resin, agitate for 30 min.
9) Filter the resin, wash with 3x15mL of NMP, 3x15mL of ACN, each with thorough mixing.
10) Dry resin under vacuum.

Having described preferred embodiments of the invention, it will now become apparent to one of skill in the art that other embodiments incorporating the concepts may be used. It is felt, therefore, that these embodiments should not be limited to disclosed embodiments but rather should be limited only by the spirit and scope of the invention.

## Claims

1. A method for forming a support bound PNA dimer, said method comprising:
a) coupling a first PNA monomer to solid support comprising an acid forming cleavable linker wherein the PNA monomer comprises an acid labile N-terminal protecting group;
b) optionally washing the solid support to remove excess first PNA monomer;
c) treating the solid support with a deprotection reagent under acidic conditions that deprotect the acid labile N-terminal protecting group;
d) washing the solid support to remove the deprotection reagent; and
e) coupling a second PNA monomer to the N-terminal amine of the first PNA monomer,
wherein the final loading of the PNA dimer on the solid support is greater than or equal to 0.08 mmol per gram.

2. The method of claim 1, wherein the first and second PNA monomers are t-boc/Z protected PNA monomers, or Mmt/Bhoc protected PNA monomers comprising the same or a different nucleobase, or the first PNA monomer is an Mmt/Bhoc protected PNA monomer and the second PNA monomer is an Fmoc/Bhoc protected PNA monomer.

3. The method of claim 1, wherein the nucleobase of the first and second PNA monomer is independently selected from the group consisting of: adenine, cytosine, guanine, thymine, uracil, 5-propynyl-uracil, 2-thio-5-propynyl-uracil, 5-methylcytosine, pseudoisocytosine, 2-thiouracil and 2-thiothymine, 2-aminopurine, N9-(2-amino-6-chloropurine), N9-(2,6-diaminopurine), hypoxanthine, N9-(7-deaza-guanine), N9-(7-deaza-8-azaguanine) and N8-(7-deaza-8-aza-adenine).

4. The method of claim 1, wherein the first PNA monomer is an Mmt/Bhoc protected PNA monomer and the deprotection reagent is a solution containing from about 1 to about 5 percent (v/v) dicloroacetic acid in an organic solvent, preferably the deprotection reagent is about 2 percent dichloroacetic acid in dichloromethane (DCM).

5. The method of claim 1, wherein the solid support is a sterically hindered solid support selected from the group consisting of: Trityl chloride resin (Trityl-Cl), 2-Chlorotrityl chloride resin, DHPP, MBHA, 4-methyltrityl chloride resin, 4-methoxytrityl chloride resin, Hydroxy-(2-chorophenyl)methyl-PS, Rink Acid Resin and NovaSyn TGT alcohol resin, or the solid support is selected from the group consisting of: Fmoc-PAL-PEG-PS, NovaSyn TGA and Wang Resin.

6. The method of claim 1, wherein the final loading of the PNA dimer on the solid support is in the range from about 0.1 mmol per gram to about 1.2 mmol per gram, preferably from about 0.12 mmol per gram to about 0.35 mmol per gram.

7. A library comprising at least two solid supports wherein said at least two solid supports each comprise:
a) an acid forming cleavable linker; and
b) a PNA dimer that: (i) is cleavably linked to the acid forming cleavable linker; and (ii) differs in nucleobase sequence from the PNA dimer that is linked to any of the other of the at least two solid supports of the library.

8. The library of claim 7, wherein the library comprises at least sixteen solid supports, each support comprising a PNA dimer chosen from a set of at least sixteen possible PNA dimers wherein each PNA dimer of the set differs from all of the other PNA dimers of the set by at least one of at least four different nucleobases.

9. The library of claim 8, wherein each of the at least four different nucleobases is selected from the group consisting of: adenine, cytosine, guanine, thymine, uracil, 5-propynyl-uracil, 2-thio-5-propynyl-uracil, 5-methylcytosine, pseudoisocytosine, 2-thiouracil and 2-thiothymine, 2-aminopurine, N9-(2-amino-6-chloropurine), N9-(2,6-diaminopurine), hypoxanthine, N9-(7-deaza-guanine), N9-(7-deaza-8-aza-guanine) and N8-(7-deaza-8-aza-adenine).

10. The library of claim 7, wherein the solid support is a sterically hindered solid support.

11. The library of claim 10, wherein the sterically hindered solid support is selected from the group consisting of: Trityl chloride resin (Trityl-Cl), 2-Chlorotrityl chloride resin, DHPP, MBHA, 4-methyltrityl chloride resin, 4-methoxytrityl chloride resin, Hydroxy-(2-chorophenyl)methyl-PS, Rink Acid Resin and NovaSyn TGT alcohol resin.

12. A PNA C-terminal acid oligomer comprising a C-terminal PNA subunit and a fluorescent label or quencher.

13. A library of PNA C-terminal acid oligomers, each PNA oligomer of the library comprising:
a) a nucleobase sequence;
b) a C-terminal PNA subunit; and
c) a fluorescent label or quencher moiety;
wherein each PNA oligomer differs, either in label, nucleobase sequence, subunit length or polarity of nucleobase sequence, from each of the other PNA oligomers of the library.

14. A solid support composition comprising:
a) an acid forming cleavable linker; and
b) a PNA dimer, comprising an N-terminal base labile protecting group, cleavably linked to the solid support through the cleavable linker,
wherein the loading of the PNA dimer on the solid support is greater than or equal to 0.08 mmol per gram.

15. A method for forming a support bound PNA dimer, said method comprising:
a) coupling a first PNA monomer to a sterically hindered solid support comprising a sterically hindered acid forming cleavable linker
wherein the PNA monomer comprises a N-terminal amine base labile protecting group;
b) optionally washing the solid support to remove excess first PNA monomer;
c) treating the solid support for a period of about 1 to about 2 minutes with a deprotection reagent that substantially removes the base labile N-terminal amine protecting group from the support bound first PNA monomer but that does not allow for more than 50 percent cyclization and elimination of the first PNA monomer from the support;
d) washing the solid support to remove the deprotection reagent; and
e) coupling a second PNA monomer to the N-terminal amine of the first PNA monomer as soon as is practical after performing steps (c) and (d).
